# EUROPEAN PATENT APPLICATION

(11) **EP 1 559 781 A1**
(43) Date of publication of application: **03.08.2005**
(21) Application number: 03810659.7
(22) Date of filing: 07.11.2003
(51) Int. Cl.: C12N 15/00, C12N 9/24, C12N 9/38, C12Q 1/68, A01K 67/00

(54) **METHOD OF DETECTING BONE PAGET'S DISEASE**

(30) Priority: 07.11.2002 JP 2002323438
(71) Applicant: National Institute of Advanced Industrial Science and Technology, Tokyo 100-8921 (JP)
(72) Inventor: NARIMATSU, Hisashi, Nat.Inst.Adv.Ind.Sci.&Tech., Tsukuba-shi, Ibaraki 305-8568 (JP); SATO, Takashi, Nat. Inst. Adv. Ind. Sci.& Tech., Tsukuba-shi, Ibaraki 305-8568 (JP); GOTOH, Masanori, Nat. Inst. Adv. Ind. Sci.&Tech., Tsukuba-shi, Ibaraki 305-8568 (JP)
(74) Representative: HOFFMANN - EITLE
(86) International application number: PCT/JP2003/014211
(87) International publication number: WO 2004/042055

(57) **Abstract**

A method for detecting Paget disease of bone and an animal showing the pathology of Paget disease of bone are provided. In particular, there are provided associating Paget disease of bone with the mutation a chondroitin/chondroitin synthase gene comprising the amino acid sequence depicted in any one of SEQ ID NO: 2, SEQ ID NO: 4, SEQ ID NO: 6, SEQ ID NO: 66, SEQ ID NO: 68 or SEQ ID NO: 70 or the amount of expression of said gene, detection of Paget disease of bone, and a knockout animal prepared by knocking out the chondroitin/chondroitin synthase gene.

SEQUENCE LISTING

<110> NATIONAL INSTITUTE OF ADVANCED INDUSTRIAL SCIENCE AND TECHNOLOGY
      AMERSHAM BIOSCIENCES K.K.

<120> METHOD OF DETECTING BONE PAGET' S DISEASE

<130> YCT-882

<150> JP2002-323438
<151> 2002-11-07

<160> 70

<170> PatentIn version 3.1

<210> 1
<211> 2649
<212> DNA
<213> Homo sapiens

<220>
<221> CDS
<222> (1)..(2649)
<223>

<400> 1
atg gct gtg cgc tct cgc cgc ccg tgg atg agc gtg gca tta ggg ctg      48
gtg ctg ggc ttc acc gcc gcg tcc tgg ctc atc gcc ccc agg gtg gcg      96
gag ctg agc gag agg aag aga cgt ggc tcc agc ctc tgc tcc tac tac      144
ggt cgc tct gct gct ggc ccc cgc gcc ggc gct cag cag ccg ctc ccc      192
cag ccc cag tcc cga cca cgg cag gag cag tcg ccg ccc ccc gcg cgc     240
cag gat ctc cag ggg cca ccg ctg ccc gag gca gca ccc ggg atc acc     288
agt ttt cga agc agc ccc tgg cag cag cca cct ccg ctg cag cag cgg     336
cgg cga gga cgc gag cct gag ggc gcg acg ggg ctt ccc ggt gct cca     384
gcg gcc gag ggg gag ccc gag gag gag gac ggg ggc gcg gct ggg cag     432
cgg aga gac ggc cgg ccg ggg agt agc cac aac ggc agc ggg gac ggg     480
ggc gct gcc gcc ccg agc gcc cga ccc cgg gac ttc ctg tac gtg ggg     528
gtg atg acc gcg cag aag tac ctg ggc agc cgc gcg ctg gcc gcg cag     576
cgg acc tgg gcg cgt ttc atc ccg ggc cgc gtg gag ttc ttt tcc agc     624
cag cag ccc ccc aac gcc ggc cag ccc ccg cca ccc ctg cct gtc atc     672
gcg cta ccg ggt gtg gac gac tcc tat cct ccc cag aaa aag tcc ttc     720
atg atg atc aag tac atg cac gac cac tac ctg gac aag tat gag tgg     768
ttc atg cgc gcc gac gac gat gtc tac atc aaa ggt gat aaa tta gaa 816
gag ttt ctt aga tcg cta aac agc agt aag cct ctc tac ctg gga cag 864
act ggc ctg ggg aat att gaa gag ctt gga aag ctg gga ctg gag cct 912
ggg gaa aac ttc tgt atg gga gga cct ggc atg atc ttt agc cga gaa 960
gtt ctc agg agg atg gtg cca cat att ggt gaa tgc ctt aga gaa atg 1008
tac acg act cat gag gat gtg gaa gta gga aga tgc gtt cgc cgt ttt 1056
ggt ggg act cag tgt gtc tgg tct tac gag atg caa caa ctg ttc cat 1104
gaa aat tat gaa cac aat cgg aag ggt tac atc caa gac ctt cac aat 1152
agc aaa atc cat gca gcc ata aca ctt cat ccc aac aaa agg cct gca 1200
tac caa tac agg ctg cat aat tac atg ctc agc cgc aaa att tct gaa 1248
ctt cgc tac cgc acc atc cag ctc cac agg gaa agt gcc ctg atg agc 1296
aag ctc agt aac aca gaa gtg agc aaa gag gac cag cag ctg gga gtg 1344
ata cct tct ttc aac cac ttc cag cct cgg gag aga aat gaa gtg ata 1392
gaa tgg gag ttc ctg aca ggg aag ctt cta tac tca gca gct gag aac 1440
cag ccc cct cga cag agc ctc agt agc att tta aga aca gca ctg gat 1488
gat acc gtc cta cag gtg atg gag atg atc aat gag aat gcc aag agc 1536
aga gga cgg ctc att gac ttc aag gaa att cag tat ggc tac cgc aga 1584
gtt aac ccc atg cac ggg gtg gag tac att ttg gat tta ctc ctt tta 1632
tac aaa aga cac aag gga agg aaa ctg act gtg cca gtg aga cgt cat 1680
gcc tat ctt cag cag ttg ttc agc aag cct ttc ttc aga gag acc gaa 1728
gag cta gat gtc aac agt ctt gtg gag agt att aac agt gaa act cag 1776
tca ttc tcc ttt ata tct aat tct tta aag ata tta tct tct ttt caa 1824
ggt gcc aaa gaa atg gga ggg cac aat gaa aag aaa gta cac att ctc 1872
gtt cct ctc atc gga agg tat gac att ttc ttg aga ttc atg gag aac 1920
ttt gaa aac atg tgt ctt atc cca aag cag aat gta aag ttg gtc att 1968
atc ctt ttc agt agg gat tct ggc caa gac tcc agc aag cat att gag 2016
ctg ata aaa ggg tac cag aac aag tac ccc aaa gca gaa atg acc ctg 2064
atc cca atg aag gga gag ttt tcc aga ggt ctt ggt ctt gaa atg gct 2112
tct gcc cag ttt gac aat gac act ttg ctg cta ttt tgt gat gtt gac 2160
ttg atc ttc aga gaa gat ttt ctc caa cga tgt aga gac aat aca att 2208
cag gga caa cag gtg tac tat ccc atc atc ttt agc cag tat gac cca 2256
aag gta aca aac ggg gga aat cct ccc act gat ggt tac ttc ata ttc 2304
tca aaa aag act gga ttt tgg aga gac tat gga tat ggc atc acc tgt 2352
att tac aaa agt gat ctt cta ggt gca ggt gga ttt gat acc tca ata 2400
caa ggc tgg gga cta gaa gat gta gat ctc tac aat aaa gtc att cta 2448
tct ggc tta agg cca ttc aga agc caa gaa gta gga gtg gtg cat att 2496
ttc cat cca gtt cat tgt gat cct aac ttg gac cct aag cag tat aag 2544
atg tgc tta gga tcc aag gca agt act ttc gcc tca acc atg caa ctg 2592
gct gaa ctc tgg ctt gaa aaa cat tta ggt gtc agg tac aat cga act 2640
ctc tcc tga 2649

<210> 2
<211> 882
<212> PRT
<213> Homo sapiens

<400> 2

<210> 3
<211> 2328
<212> DNA
<213> Homo sapiens

<220>
<221> CDS
<222> (1)..(2328)
<223>

<400> 3
atg cgg gca tcg ctg ctg ctg tcg gtg ctg cgg ccc gca ggg ccc gtg 48
Met Arg Ala Ser Leu Leu Leu Ser Val Leu Arg Pro Ala Gly Pro Val
1               5                   10                  15
gcc gtg ggc atc tcc ctg ggc ttc acc ctg agc ctg ctc agc gtc acc 96
Ala Val Gly Ile Ser Leu Gly Phe Thr Leu Ser Leu Leu Ser Val Thr
            20                  25                  30
tgg gtg gag gag ccg tgc ggc cca ggc ccg ccc caa cct gga gac tct 144

Trp Val Glu Glu Pro Cys Gly Pro Gly Pro Pro Gln Pro Gly Asp Ser
        35                  40                  45
gag ctg ccg ccg cgc ggc aac acc aac gcg gcg cgc cgg ccc aac tcg 192
Glu Leu Pro Pro Arg Gly Asn Thr Asn Ala Ala Arg Arg Pro Asn Ser
    50                  55                  60
gtg cag ccc gga gcg gag cgc gag aag ccc ggg gcc ggc gaa ggc gcc 240
Val Gln Pro Gly Ala Glu Arg Glu Lys Pro Gly Ala Gly Glu Gly Ala
65                  70                  75                  80
ggg gag aat tgg gag ccg cgc gtc ttg ccc tac cac cct gca cag ccc 288
Gly Glu Asn Trp Glu Pro Arg Val Leu Pro Tyr His Pro Ala Gln Pro
                85                  90                  95
ggc cag gcc gcc aaa aag gcc gtc agg acc cgc tac atc agc acg gag 336
Gly Gln Ala Ala Lys Lys Ala Val Arg Thr Arg Tyr Ile Ser Thr Glu
            100                 105                 110
ctg ggc atc agg cag agg ctg ctg gtg gcg gtg ctg acc tct cag acc 384
Leu Gly Ile Arg Gln Arg Leu Leu Val Ala Val Leu Thr Ser Gln Thr
        115                 120                 125
acg ctg ccc acg ctg ggc gtg gcc gtg aac cgc acg ctg ggg cac cgg 432
Thr Leu Pro Thr Leu Gly Val Ala Val Asn Arg Thr Leu Gly His Arg
    130                 135                 140
ctg gag cgt gtg gtg ttc ctg acg ggc gca cgg ggc cgc cgg gcc cca 480
Leu Glu Arg Val Val Phe Leu Thr Gly Ala Arg Gly Arg Arg Ala Pro
145                 150                 155                 160
cct ggc atg gca gtg gtg acg ctg ggc gag gag cga ccc att gga cac 528
Pro Gly Met Ala Val Val Thr Leu Gly Glu Glu Arg Pro Ile Gly His
                165                 170                 175
ctg cac ctg gcg ctg cgc cac ctg ctg gag cag cac ggc gac gac ttt 576
Leu His Leu Ala Leu Arg His Leu Leu Glu Gln His Gly Asp Asp Phe
            180                 185                 190
gac tgg ttc ttc ctg gtg cct gac acc acc tac acc gag gcg cac ggc 624
Asp Trp Phe Phe Leu Val Pro Asp Thr Thr Tyr Thr Glu Ala His Gly
        195                 200                 205
ctg gca cgc cta act ggc cac ctc agc ctg gcc tcc gcc gcc cac ctg 672
Leu Ala Arg Leu Thr Gly His Leu Ser Leu Ala Ser Ala Ala His Leu
    210                 215                 220
tac ctg ggc cgg ccc cag gac ttc atc ggc gga gag ccc acc ccc ggc 720
Tyr Leu Gly Arg Pro Gln Asp Phe Ile Gly Gly Glu Pro Thr Pro Gly

225                 230                 235                 240
cgc tac tgc cac gga ggc ttt ggg gtg ctg ctg tcg cgc atg ctg ctg     768
Arg Tyr Cys His Gly Gly Phe Gly Val Leu Leu Ser Arg Met Leu Leu
                245                 250                 255
caa caa ctg cgc ccc cac ctg gaa ggc tgc cgc aac gac atc gtc agt     816
Gln Gln Leu Arg Pro His Leu Glu Gly Cys Arg Asn Asp Ile Val Ser
            260                 265                 270
gcg cgc cct gac gag tgg ctg ggt cgc tgc att ctc gat gcc acc ggg     864
Ala Arg Pro Asp Glu Trp Leu Gly Arg Cys Ile Leu Asp Ala Thr Gly
        275                 280                 285
gtg ggc tgc act ggt gac cac gag ggg gtg cac tat agc cat ctg gag     912
Val Gly Cys Thr Gly Asp His Glu Gly Val His Tyr Ser His Leu Glu
    290                 295                 300
ctg agc cct ggg gag cca gtg cag gag ggg gac cct cat ttc cga agt     960
Leu Ser Pro Gly Glu Pro Val Gln Glu Gly Asp Pro His Phe Arg Ser
305                 310                 315                 320
gcc ctg aca gcc cac cct gtg cgt gac cct gtg cac atg tac cag ctg     1008
Ala Leu Thr Ala His Pro Val Arg Asp Pro Val His Met Tyr Gln Leu
                325                 330                 335
cac aaa gct ttc gcc cga gct gaa ctg gaa cgc acg tac cag gag atc     1056
His Lys Ala Phe Ala Arg Ala Glu Leu Glu Arg Thr Tyr Gln Glu Ile
            340                 345                 350
cag gag tta cag tgg gag atc cag aat acc agc cat ctg gcc gtt gat     1104
Gln Glu Leu Gln Trp Glu Ile Gln Asn Thr Ser His Leu Ala Val Asp
        355                 360                 365
ggg gac cgg gca gct gct tgg ccc gtg ggt att cca gca cca tcc cgc     1152
Gly Asp Arg Ala Ala Ala Trp Pro Val Gly Ile Pro Ala Pro Ser Arg
    370                 375                 380
ccg gcc tcc cgc ttt gag gtg ctg cgc tgg gac tac ttc acg gag cag     1200
Pro Ala Ser Arg Phe Glu Val Leu Arg Trp Asp Tyr Phe Thr Glu Gln
385                 390                 395                 400
cac gct ttc tcc tgc gcc gat ggc tca ccc cgc tgc cca ctg cgt ggg     1248
His Ala Phe Ser Cys Ala Asp Gly Ser Pro Arg Cys Pro Leu Arg Gly
                405                 410                 415
gct gac cgg gct gat gtg gcc gat gtt ctg ggg aca gct cta gag gag     1296
Ala Asp Arg Ala Asp Val Ala Asp Val Leu Gly Thr Ala Leu Glu Glu
            420                 425                 430

ctg aac cgc cgc tac cac ccg gcc ttg cgg ctc cag aag cag cag ctg 1344
Leu Asn Arg Arg Tyr His Pro Ala Leu Arg Leu Gln Lys Gln Gln Leu
        435                 440                 445
gtg aat ggc tac cga cgc ttt gat ccg gcc cgg ggt atg gaa tac acg 1392
Val Asn Gly Tyr Arg Arg Phe Asp Pro Ala Arg Gly Met Glu Tyr Thr
    450                 455                 460
ctg gac ttg cag ctg gag gca ctg acc ccc cag gga ggc cgc cgg ccc 1440
Leu Asp Leu Gln Leu Glu Ala Leu Thr Pro Gln Gly Gly Arg Arg Pro
465                 470                 475                 480
ctc act cgc cga gtg cag ctg ctc cgg ccg ctg agc cgc gtg gag atc 1488
Leu Thr Arg Arg Val Gln Leu Leu Arg Pro Leu Ser Arg Val Glu Ile
                485                 490                 495
ttg cct gtg ccc tat gtc act gag gcc tca cgt ctc act gtg ctg ctg 1536
Leu Pro Val Pro Tyr Val Thr Glu Ala Ser Arg Leu Thr Val Leu Leu
            500                 505                 510
cct cta gct gcg gct gag cgt gac ctg gcc cct ggc ttc ttg gag gcc 1584
Pro Leu Ala Ala Ala Glu Arg Asp Leu Ala Pro Gly Phe Leu Glu Ala
        515                 520                 525
ttt gcc act gca gca ctg gag cct ggt gat gct gcg gca gcc ctg acc 1632
Phe Ala Thr Ala Ala Leu Glu Pro Gly Asp Ala Ala Ala Ala Leu Thr
    530                 535                 540
ctg ctg cta ctg tat gag ccg cgc cag gcc cag cgc gtg gcc cat gca 1680
Leu Leu Leu Leu Tyr Glu Pro Arg Gln Ala Gln Arg Val Ala His Ala
545                 550                 555                 560
gat gtc ttc gca cct gtc aag gcc cac gtg gca gag ctg gag cgg cgt 1728
Asp Val Phe Ala Pro Val Lys Ala His Val Ala Glu Leu Glu Arg Arg
                565                 570                 575
ttc ccc ggt gcc cgg gtg cca tgg ctc agt gtg cag aca gcc gca ccc 1776
Phe Pro Gly Ala Arg Val Pro Trp Leu Ser Val Gln Thr Ala Ala Pro
            580                 585                 590
tca cca ctg cgc ctc atg gat cta ctc tcc aag aag cac ccg ctg gac 1824
Ser Pro Leu Arg Leu Met Asp Leu Leu Ser Lys Lys His Pro Leu Asp
        595                 600                 605
aca ctg ttc ctg ctg gcc ggg cca gac acg gtg ctc acg cct gac ttc 1872
Thr Leu Phe Leu Leu Ala Gly Pro Asp Thr Val Leu Thr Pro Asp Phe
    610                 615                 620
ctg aac cgc tgc cgc atg cat gcc atc tcc ggc tgg cag gcc ttc ttt 1920

Leu Asn Arg Cys Arg Met His Ala Ile Ser Gly Trp Gln Ala Phe Phe
625                 630                 635                 640
ccc atg cat ttc caa gcc ttc cac cca gct gtg gcc cca cca caa ggg      1968
Pro Met His Phe Gln Ala Phe His Pro Ala Val Ala Pro Pro Gln Gly
                645                 650                 655
cct ggg ccc cca gag ctg ggc cgt gac act ggc cgc ttt gat cgc cag      2016
Pro Gly Pro Pro Glu Leu Gly Arg Asp Thr Gly Arg Phe Asp Arg Gln
            660                 665                 670
gca gcc agc gag gcc tgc ttc tac aac tcc gac tac gtg gca gcc cgt      2064
Ala Ala Ser Glu Ala Cys Phe Tyr Asn Ser Asp Tyr Val Ala Ala Arg
        675                 680                 685
ggg cgc ctg gcg gca gcc tca gaa caa gaa gag gag ctg ctg gag agc      2112
Gly Arg Leu Ala Ala Ala Ser Glu Gln Glu Glu Glu Leu Leu Glu Ser
    690                 695                 700
ctg gat gtg tac gag ctg ttc ctc cac ttc tcc agt ctg cat gtg ctg      2160
Leu Asp Val Tyr Glu Leu Phe Leu His Phe Ser Ser Leu His Val Leu
705                 710                 715                 720
cgg gcg gtg gag ccg gcg ctg ctg cag cgc tac cgg gcc cag acg tgc      2208
Arg Ala Val Glu Pro Ala Leu Leu Gln Arg Tyr Arg Ala Gln Thr Cys
                725                 730                 735
agc gcg agg ctc agt gag gac ctg tac cac cgc tgc ctc cag agc gtg      2256
Ser Ala Arg Leu Ser Glu Asp Leu Tyr His Arg Cys Leu Gln Ser Val
            740                 745                 750
ctt gag ggc ctc ggc tcc cga acc cag ctg gcc atg cta ctc ttt gaa      2304
Leu Glu Gly Leu Gly Ser Arg Thr Gln Leu Ala Met Leu Leu Phe Glu
        755                 760                 765
cag gag cag ggc aac agc acc tga                                      2328
Gln Glu Gln Gly Asn Ser Thr
    770                 775

<210>  4
<211>  775
<212>  PRT
<213>  Homo sapiens

<400>  4

Met Arg Ala Ser Leu Leu Leu Ser Val Leu Arg Pro Ala Gly Pro Val
1               5                   10                  15

Ala Val Gly Ile Ser Leu Gly Phe Thr Leu Ser Leu Leu Ser Val Thr
            20                  25                  30

Trp Val Glu Glu Pro Cys Gly Pro Gly Pro Pro Gln Pro Gly Asp Ser
        35                  40                  45

Glu Leu Pro Pro Arg Gly Asn Thr Asn Ala Ala Arg Arg Pro Asn Ser
    50                  55                  60

Val Gln Pro Gly Ala Glu Arg Glu Lys Pro Gly Ala Gly Glu Gly Ala
65                  70                  75                  80

Gly Glu Asn Trp Glu Pro Arg Val Leu Pro Tyr His Pro Ala Gln Pro
                85                  90                  95

Gly Gln Ala Ala Lys Lys Ala Val Arg Thr Arg Tyr Ile Ser Thr Glu
            100                 105                 110

Leu Gly Ile Arg Gln Arg Leu Leu Val Ala Val Leu Thr Ser Gln Thr
        115                 120                 125

Thr Leu Pro Thr Leu Gly Val Ala Val Asn Arg Thr Leu Gly His Arg
    130                 135                 140

Leu Glu Arg Val Val Phe Leu Thr Gly Ala Arg Gly Arg Arg Ala Pro

145                 150                 155                 160

Pro Gly Met Ala Val Val Thr Leu Gly Glu Glu Arg Pro Ile Gly His
                165                 170                 175

Leu His Leu Ala Leu Arg His Leu Leu Glu Gln His Gly Asp Asp Phe
            180                 185                 190

Asp Trp Phe Phe Leu Val Pro Asp Thr Thr Tyr Thr Glu Ala His Gly
        195                 200                 205

Leu Ala Arg Leu Thr Gly His Leu Ser Leu Ala Ser Ala Ala His Leu
    210                 215                 220

Tyr Leu Gly Arg Pro Gln Asp Phe Ile Gly Gly Glu Pro Thr Pro Gly
225                 230                 235                 240

Arg Tyr Cys His Gly Gly Phe Gly Val Leu Leu Ser Arg Met Leu Leu
                245                 250                 255

Gln Gln Leu Arg Pro His Leu Glu Gly Cys Arg Asn Asp Ile Val Ser
            260                 265                 270

Ala Arg Pro Asp Glu Trp Leu Gly Arg Cys Ile Leu Asp Ala Thr Gly
        275                 280                 285

Val Gly Cys Thr Gly Asp His Glu Gly Val His Tyr Ser His Leu Glu
    290                 295                 300

Leu Ser Pro Gly Glu Pro Val Gln Glu Gly Asp Pro His Phe Arg Ser
305                 310                 315                 320

Ala Leu Thr Ala His Pro Val Arg Asp Pro Val His Met Tyr Gln Leu
                325                 330                 335

His Lys Ala Phe Ala Arg Ala Glu Leu Glu Arg Thr Tyr Gln Glu Ile
            340                 345                 350

Gln Glu Leu Gln Trp Glu Ile Gln Asn Thr Ser His Leu Ala Val Asp
        355                 360                 365

Gly Asp Arg Ala Ala Ala Trp Pro Val Gly Ile Pro Ala Pro Ser Arg
    370                 375                 380

Pro Ala Ser Arg Phe Glu Val Leu Arg Trp Asp Tyr Phe Thr Glu Gln
385                 390                 395                 400

His Ala Phe Ser Cys Ala Asp Gly Ser Pro Arg Cys Pro Leu Arg Gly
                405                 410                 415

Ala Asp Arg Ala Asp Val Ala Asp Val Leu Gly Thr Ala Leu Glu Glu
            420                 425                 430

Leu Asn Arg Arg Tyr His Pro Ala Leu Arg Leu Gln Lys Gln Gln Leu
        435                 440                 445

Val Asn Gly Tyr Arg Arg Phe Asp Pro Ala Arg Gly Met Glu Tyr Thr
    450                 455                 460

Leu Asp Leu Gln Leu Glu Ala Leu Thr Pro Gln Gly Gly Arg Arg Pro
465                 470                 475                 480

Leu Thr Arg Arg Val Gln Leu Leu Arg Pro Leu Ser Arg Val Glu Ile
                485                 490                 495

Leu Pro Val Pro Tyr Val Thr Glu Ala Ser Arg Leu Thr Val Leu Leu
            500                 505                 510

Pro Leu Ala Ala Ala Glu Arg Asp Leu Ala Pro Gly Phe Leu Glu Ala
        515                 520                 525

Phe Ala Thr Ala Ala Leu Glu Pro Gly Asp Ala Ala Ala Ala Leu Thr
    530                 535                 540

Leu Leu Leu Leu Tyr Glu Pro Arg Gln Ala Gln Arg Val Ala His Ala
545                 550                 555                 560

Asp Val Phe Ala Pro Val Lys Ala His Val Ala Glu Leu Glu Arg Arg
                565                 570                 575

Phe Pro Gly Ala Arg Val Pro Trp Leu Ser Val Gln Thr Ala Ala Pro
            580                 585                 590

Ser Pro Leu Arg Leu Met Asp Leu Leu Ser Lys Lys His Pro Leu Asp
        595                 600                 605

Thr Leu Phe Leu Leu Ala Gly Pro Asp Thr Val Leu Thr Pro Asp Phe
    610                 615                 620

Leu Asn Arg Cys Arg Met His Ala Ile Ser Gly Trp Gln Ala Phe Phe
625                 630                 635                 640

Pro Met His Phe Gln Ala Phe His Pro Ala Val Ala Pro Pro Gln Gly
                645                 650                 655

Pro Gly Pro Pro Glu Leu Gly Arg Asp Thr Gly Arg Phe Asp Arg Gln
            660                 665                 670

Ala Ala Ser Glu Ala Cys Phe Tyr Asn Ser Asp Tyr Val Ala Ala Arg
        675                 680                 685

Gly Arg Leu Ala Ala Ala Ser Glu Gln Glu Glu Glu Leu Leu Glu Ser
    690                 695                 700

Leu Asp Val Tyr Glu Leu Phe Leu His Phe Ser Ser Leu His Val Leu
705                 710                 715                 720

Arg Ala Val Glu Pro Ala Leu Leu Gln Arg Tyr Arg Ala Gln Thr Cys
                725                 730                 735

Ser Ala Arg Leu Ser Glu Asp Leu Tyr His Arg Cys Leu Gln Ser Val

            740                 745                 750

Leu Glu Gly Leu Gly Ser Arg Thr Gln Leu Ala Met Leu Leu Phe Glu
        755                 760                 765

Gln Glu Gln Gly Asn Ser Thr
    770                 775

<210> 5
<211> 1669
<212> DNA
<213> Homo sapiens

<220>
<221> CDS
<222> (41)..(1024)
<223>

<400> 5
ctgcgagcgc ctgccccatg cgccgccgcc tctccgcacg atg ttc ccc tcg cgg 55
agg aaa gcg gcg cag ctg ccc tgg gag gac ggc agg tcc ggg ttg ctc 103
tcc ggc ggc ctc cct cgg aag tgt tcc gtc ttc cac ctg ttc gtg gcc 151
tgc ctc tcg ctg ggc ttc ttc tcc cta ctc tgg ctg cag ctc agc tgc 199
tct ggg gac gtg gcc cgg gca gtc agg gga caa ggg cag gag acc tcg 247
ggc cct ccc cgc gcc tgc ccc cca gag ccg ccc cct gag cac tgg gaa 295
gaa gac gca tcc tgg ggc ccc cac cgc ctg gca gtg ctg gtg ccc ttc 343
cgc gaa cgc ttc gag gag ctc ctg gtc ttc gtg ccc cac atg cgc cgc 391
ttc ctg agc agg aag aag atc cgg cac cac atc tac gtg ctc aac cag 439
gtg gac cac ttc agg ttc aac cgg gca gcg ctc atc aac gtg ggc ttc 487
ctg gag agc agc aac agc acg gac tac att gcc atg cac gac gtt gac 535
ctg ctc cct ctc aac gag gag ctg gac tat ggc ttt cct gag gct ggg 583
ccc ttc cac gtg gcc tcc ccg gag ctc cac cct ctc tac cac tac aag 631
acc tat gtc ggc ggc atc ctg ctg ctc tcc aag cag cac tac cgg ctg 679
tgc aat ggg atg tcc aac cgc ttc tgg ggc tgg ggc cgc gag gac gac 727
gag ttc tac cgg cgc att aag gga gct ggg ctc cag ctt ttc cgc ccc 775
tcg gga atc aca act ggg tac aag aca ttt cgc cac ctg cac gac cca 823
gcc tgg cgg aag agg gac cag aag cgc atc gca gct caa aaa cag gag 871
cag ttc aag gtg gac agg gag gga ggc ctg aac act gtg aag tac cat      919
gtg gct tcc cgc act gcc ctg tct gtg ggc ggg gcc ccc tgc act gtc      967
ctc aac atc atg ttg gac tgt gac aag acc gcc aca ccc tgg tgc aca      1015
ttc agc tga gctggatgga cagtgaggaa gcctgtacct acaggccata              1064
ttgctcaggc tcaggacaag gcctcaggtc gtgggcccag ctctgacagg atgtggagtg    1124
gccaggacca agacagcaag ctacgcaatt gcagccaccc ggccgccaag gcaggcttgg    1184
gctgggccag gacacgtggg gtgcctggga cgctgcttgc catgcacagt gatcagagag    1244
aggctggggt gtgtcctgtc cgggaccccc cctgccttcc tgctcaccct actctgacct    1304
ccttcacgtg cccaggcctg tgggtagtgg ggagggctga acaggacaac ctctcatcac    1364
ccccactttt gttccttcct gctgggctgc ctcgtgcaga gacacagtgt aggggccatg    1424
cagctggcgt aggtggcagt tgggcctggt gagggttagg acttcagaaa ccagagcaca    1484
agccccacag agggggaaca gccagcaccg ctctagctgg ttgttgccat gccggaatgt    1544
gggcctagtg ttgccagatc ttctgatttt tcgaaagaaa ctagaatgct ggattcttaa    1604
gtgatatctt ctgatttttt aaatgatagc acctaaatga aactttcaaa aagtaaaaaa    1664
aaaaa                                                                1669

<210> 6
<211> 327
<212> PRT
<213> Homo sapiens

<400> 6

<210> 7
<211> 26
<212> DNA
<213> Artificial

<220>
<223> Description of Artificial Sequence: 5' primer for PCR (K3)

<400> 7
cccaagcttg ccgaggggga gcccga                                          26

<210> 8
<211> 29
<212> DNA
<213> Artificial

<220>
<223> Description of Artificial Sequence: 3' primer for PCR (K3)

<400> 8
gctctagact gtcaggagag agttcgatt                                       29

<210> 9
<211> 25
<212> DNA
<213> Artificial

<220>
<223> Description of Artificial Sequence: 5' primer for PCR (K3)

<400> 9
atggctgtgc gctctcgccg cccgt                                           25

<210> 10
<211> 25
<212> DNA
<213> Artificial

<220>
<223> Description of Artificial Sequence: 3' primer for PCR (K3)

<400> 10
cgtccccgct gccgttgtgg ctact 25

<210> 11
<211> 25
<212> DNA
<213> Artificial

<220>
<223> Description of Artificial Sequence: 5' primer for PCR (K3)

<400> 11
agtagccaca acggcagcgg ggacg 25

<210> 12
<211> 24
<212> DNA
<213> Artificial

<220>
<223> Description of Artificial Sequence: 3' primer for PCR (K3)

<400> 12
tcaggagaga gttcgattgt acct 24

<210> 13
<211> 28
<212> DNA

<213>  Artificial

<220>
<223>  Description of Artificial Sequence: 5' primer for PCR (K11)

<400>  13
ggaattccgg ccaggccgcc aaaaaggc                                          28

<210>  14
<211>  30
<212>  DNA
<213>  Artificial

<220>
<223>  Description of Artificial Sequence: 3' primer for PCR (K11)

<400>  14
cgggatcctc aggtgctgtt gccctgctcc                                        30

<210>  15
<211>  16
<212>  PRT
<213>  Artificial

<220>
<223>  Description of Artificial Sequence: synthesised substance for ass
ay

<400>  15

Val Leu Pro Gln Glu Glu Glu Gly Ser Gly Gly Gly Gln Leu Val Thr
1               5                   10                  15


<210>  16
<211>  20
<212>  DNA

<213>  Artificial

<220>
<223>  Description of Artificial Sequence: 5' primer for PCR and sequenc
ing (K3 exon 1)

<400>  16
cgacagccca gcgagcgtcc                                                    20

<210>  17
<211>  22
<212>  DNA
<213>  Artificial

<220>
<223>  Description of Artificial Sequence: 3' primer for PCR and sequenc
ing (K3 exon 1)

<400>  17
ggagactggc aggctggaaa gc                                                 22

<210>  18
<211>  20
<212>  DNA
<213>  Artificial

<220>
<223>  Description of Artificial Sequence: Primer for sequencing (K3 exo
n 1)

<400>  18
agggggagcc cgaggaggag                                                    20

<210>  19
<211>  20
<212>  DNA
<213>  Artificial

<220>
<223> Description of Artificial Sequence: Primer for sequencing (K3 exo
n 1)

<400> 19
ctcctcctcg ggctccccct 20

<210> 20
<211> 27
<212> DNA
<213> Artificial

<220>
<223> Description of Artificial Sequence: 5' primer for PCR and sequenc
ing (K3 exon 2)

<400> 20
gagacatagt aattgttgcc tttcttt 27

<210> 21
<211> 25
<212> DNA
<213> Artificial

<220>
<223> Description of Artificial Sequence: 3' primer for PCR and sequenc
ing (K3 exon 2)

<400> 21
gtgaacattt tcatcacagc tccat 25

<210> 22
<211> 26
<212> DNA
<213> Artificial

<220>
<223> Description of Artificial Sequence: 5' primer for PCR and sequenc
ing (K3 exon 3)

<400> 22
tagatgcttt agtttatcgc tggttt 26

<210> 23
<211> 26
<212> DNA
<213> Artificial

<220>
<223> Description of Artificial Sequence: 3' primer for PCR and sequenc
ing (K3 exon 3)

<400> 23
ttaaaaaagg caaaatgtgt tgcctg 26

<210> 24
<211> 24
<212> DNA
<213> Artificial

<220>
<223> Description of Artificial Sequence: Primer for sequencing (K3 exo
n 3)

<400> 24
tctatactca gcagctgaga acca 24

<210> 25
<211> 24
<212> DNA
<213> Artificial

<220>

<223>  Description of Artificial Sequence: Primer for sequencing (K3 exo
n 3)

<400>  25
tggttctcag ctgctgagta taga                                              24

<210>  26
<211>  24
<212>  DNA
<213>  Artificial

<220>
<223>  Description of Artificial Sequence: Primer for sequencing (K3 exo
n 3)

<400>  26
gaaatgggag ggcacaatga aaag                                              24

<210>  27
<211>  24
<212>  DNA
<213>  Artificial

<220>
<223>  Description of Artificial Sequence: Primer for sequencing (K3 exo
n 3)

<400>  27
cttttcattg tgccctccca tttc                                              24

<210>  28
<211>  24
<212>  DNA
<213>  Artificial

<220>
<223>  Description of Artificial Sequence: Primer for sequencing (K3 exo

n 3)

<400> 28
tagccagtat gacccaaagg taac 24

<210> 29
<211> 24
<212> DNA
<213> Artificial

<220>
<223> Description of Artificial Sequence: Primer for sequencing (K3 exo
n 3)

<400> 29
gttacctttg ggtcatactg gcta 24

<210> 30
<211> 31
<212> DNA
<213> Artificial

<220>
<223> Description of Artificial Sequence: Primer for sequencing (K3 exo
n 3)

<400> 30
aggccattca gaagccaaga agtaggagtg g 31

<210> 31
<211> 31
<212> DNA
<213> Artificial

<220>
<223> Description of Artificial Sequence: Primer for sequencing (K3 exo
n 3)

<400> 31
ccactcctac ttcttggctt ctgaatggcc t 31

<210> 32
<211> 21
<212> DNA
<213> Artificial

<220>
<223> Description of Artificial Sequence: 5' primer for PCR and sequenc
ing (K11 exon 1)

<400> 32
tcggagactc ctctggctgc t 21

<210> 33
<211> 21
<212> DNA
<213> Artificial

<220>
<223> Descriprion of Artificial Sequence: 3' primer for PCR and sequenc
ing (K11 exon 1)

<400> 33
tagagcgggc gcagccgatc a 21

<210> 34
<211> 25
<212> DNA
<213> Artificial

<220>
<223> Description of Artificial Sequence: 5' primer for PCR and sequenc
ing (K11 exon 2)

<400>  34
tttgataagc ttgtgccatc tcctc                                          25

<210>  35
<211>  25
<212>  DNA
<213>  Artificial

<220>
<223>  Description of Artificial Sequence: 3' primer for PCR and sequenc
ing (K11 exon 2)

<400>  35
aggtatcagt gggatagctt atcat                                          25

<210>  36
<211>  24
<212>  DNA
<213>  Artificial

<220>
<223>  Description of Artificial Sequence: 5' primer for PCR and sequenc
ing (K11 exon 3)

<400>  36
agctcatcac agatcccttc cctt                                           24

<210>  37
<211>  22
<212>  DNA
<213>  Artificial

<220>
<223>  Description of Artificial Sequence: 3' primer for PCR and sequenc
ing (K11 exon 1)

<400>  37

actctgccac ccccagacct ag 22

<210> 38
<211> 24
<212> DNA
<213> Artificial

<220>
<223> Description of Artificial Sequence: 5' primer for PCR and sequenc
ing (K11 exon 4)

<400> 38
ttgctgatgg cctgtttctc tgat 24

<210> 39
<211> 21
<212> DNA
<213> Artificial

<220>
<223> Description of Artificial Sequence: 3' primer for PCR and sequenc
ing (K11 exon 4)

<400> 39
gtgtggccat gccacggccc a 21

<210> 40
<211> 23
<212> DNA
<213> Artificial

<220>
<223> Description of Artificial Sequence: Primer for sequencing (K11 ex
on 4)

<400> 40
tatgtcactg aggcctcacg tct 23

<210> 41
<211> 23
<212> DNA
<213> Artificial

<220>
<223> Description of Artificial Sequence: Primer for sequencing (K11 ex
on 4)

<400> 41
agacgtgagg cctcagtgac ata 23

<210> 42
<211> 24
<212> DNA
<213> Artificial

<220>
<223> Description of Artificial Sequence: primer for sequencing (K11 ex
on 4)

<400> 42
atgcatttcc aagccttcca ccca 24

<210> 43
<211> 24
<212> DNA
<213> Artificial

<220>
<223> Description of Artificial Sequence: Primer for sequencing (K11 ex
on 4)

<400> 43
tgggtggaag gcttggaaat gcat 24

<210> 44
<211> 20
<212> DNA
<213> Artificial

<220>
<223> Description of Artificial Sequence: 5' primer for PCR and sequenc
ing (beta4Gal-T7 exon 1)

<400> 44
tgcgagcgcc tgccccatgc 20

<210> 45
<211> 22
<212> DNA
<213> Artificial

<220>
<223> Description of Artificial Sequence: 3' primer for PCR and sequenc
ing (beta4Gal-T7 exon 1)

<400> 45
gatggcctcg ggttcccaga tt 22

<210> 46
<211> 21
<212> DNA
<213> Artificial

<220>
<223> Description of Artificial Sequence: 5' primer for PCR and sequenc
ing (beta4Gal-T7 exon 2)

<400> 46
   tcctgaccct gtcccgcgct t 21

<210> 47

<211>  20
<212>  DNA
<213>  Artificial

<220>
<223>  Description of Artificial Sequence: 3' primer for PCR and sequenc
ing (beta4Gal-T7 exon 2)

<400>  47
aggggtgccg aggggagagg                                                    20

<210>  48
<211>  21
<212>  DNA
<213>  Artificial

<220>
<223>  Description of Artificial Sequence: 5' primer for PCR and sequenc
ing (beta4Gal-T7 exon 3)

<400>  48
ctgcccagcc ttgcccaccc t                                                  21

<210>  49
<211>  22
<212>  DNA
<213>  Artificial

<220>
<223>  Description of Artificial Sequence: 3' primer for PCR and sequenc
ing (beta4Gal-T7 exon 3)

<400>  49
gctctgagca gagcaggctg tc                                                 22

<210>  50
<211>  22

<212>  DNA
<213>  Artificial

<220>
<223>  Description of Artificial Sequence: 5' primer for PCR and sequenc
ing (beta4Gal-T7 exon 4)

<400>  50
agatgggccg agtgacgctg ct                                                22

<210>  51
<211>  21
<212>  DNA
<213>  Artificial

<220>
<223>  Description of Artificial Sequence: 3' primer for PCR and sequenc
ing (beta4Gal-T7 exon 4)

<400>  51
ctcagggcag ccaccgcagc t                                                 21

<210>  52
<211>  22
<212>  DNA
<213>  Artificial

<220>
<223>  Description of Artificial Sequence: 5' primer for PCR and sequenc
ing (beta4Gal-T7 exon 5)

<400>  52
aagggcagcc tgaccccgac tt                                                22

<210>  53
<211>  23
<212>  DNA

<213>  Artificial

<220>
<223>  Description of Artificial Sequence: 3' primer for PCR and sequenc
ing (beta4Gal-T7 exon 5)

<400>  53
atgaccacct atccgtcccc aat                                              23

<210>  54
<211>  22
<212>  DNA
<213>  Artificial

<220>
<223>  Description of Artificial Sequence: 5' primer for PCR and sequenc
ing (beta4Gal-T7 exon 6)

<400>  54
cagccctgag tccgtgctct tt                                               22

<210>  55
<211>  23
<212>  DNA
<213>  Artificial

<220>
<223>  Description of Artificial Sequence: 3' primer for PCR and sequenc
ing (beta4Gal-T7 exon 6)

<220>
<221>  misc_feature
<223>  Description of Artificial Sequence: 3' primer for PCR and sequenc
ing (beta4Gal-T7 exon 6)


<400>  55

tggcctgtag gtacaggctt cct 23

<210> 56
<211> 23
<212> DNA
<213> Artificial

<220>
<223> Description of Artificial Sequence: 5' primer for RT-PCR (K3)

<400> 56
cccagaaaaa gtccttcatg atg 23

<210> 57
<211> 30
<212> DNA
<213> Artificial

<220>
<223> Description of Artificial Sequence: 3' primer for RT-PCR (K3)

<400> 57
aactcttcta atttgtcacc tttgatgtag 30

<210> 58
<211> 17
<212> DNA
<213> Artificial

<220>
<223> Description of Artificial Sequence: Probe for RT-PCR (K3)

<400> 58
atgagtggtt catgcgc 17

<210> 59
<211> 20

<212>  DNA
<213>  Artificial

<220>
<223>  Description of Artificial Sequence: 5' primer for RT-PCR (K11)

<400>  59
gctgaactgg aacgcacgta                                                    20

<210>  60
<211>  19
<212>  DNA
<213>  Artificial

<220>
<223>  Description of Artificial Sequence: 3' primer for RT-PCR (K11)

<400>  60
cgggatggtg ctggaatac                                                     19

<210>  61
<211>  20
<212>  DNA
<213>  Artificial

<220>
<223>  Description of Artificial Sequence: Probe for RT-PCR (K11)

<400>  61
agatccagga gttacagtgg                                                    20

<210>  62
<211>  18
<212>  DNA
<213>  Artificial

<220>

<223> Description of Artificial Sequence: 5' primer for RT-PCR (beta4Ga
l-T7)

<400> 62
cggcgcatta agggagct 18

<210> 63
<211> 21
<212> DNA
<213> Artificial

<220>
<223> Description of Artificial Sequence: 3' primer for RT-PCR (beta4Ga
l-T7)

<400> 63
tacccagttg tgattcccga g 21

<210> 64
<211> 14
<212> DNA
<213> Artificial

<220>
<223> Description of Artificial Sequence: Probe for RT-PCR (beta4Gal-
T7 )

<400> 64
ctccagcttt tccg 14

<210> 65
<211> 2652
<212> DNA
<213> mouse

<220>
<221> CDS

<222>  (1)..(2652)
<223>

<400>  65
atg gcc gtg cgc tcc cgc cgc cca tgg gtg agc gtg gca ttg ggg ttg      48
Met Ala Val Arg Ser Arg Arg Pro Trp Val Ser Val Ala Leu Gly Leu
1               5                   10                  15
gtc ctg ggc ttc acc gcc gcg tcc tgg ctc atc gcc ccc cgg gtg gcc      96
Val Leu Gly Phe Thr Ala Ala Ser Trp Leu Ile Ala Pro Arg Val Ala
            20                  25                  30
gag ctg agc gag aag agg cga cgc ggc tcc agt ctt tgc tcc tac tac      144
Glu Leu Ser Glu Lys Arg Arg Arg Gly Ser Ser Leu Cys Ser Tyr Tyr
        35                  40                  45
ggc cgc tcg gct acc ggg ccc cgc gcg gac gcg cag cag ctg ctc ccc      192
Gly Arg Ser Ala Thr Gly Pro Arg Ala Asp Ala Gln Gln Leu Leu Pro
    50                  55                  60
caa ccc cag tcc cgg ccg cgg cta gag cag tcg ccg ccc cct gcc agc      240
Gln Pro Gln Ser Arg Pro Arg Leu Glu Gln Ser Pro Pro Pro Ala Ser
65                  70                  75                  80
cac gag ctc ccc ggt cct cag cag ccg gag gcg gcg ccc gga ggt ccc      288
His Glu Leu Pro Gly Pro Gln Gln Pro Glu Ala Ala Pro Gly Gly Pro
                85                  90                  95
agt ttt cgg agc agc ccc tgg cag cag ccg gct ctg ttg ccg cag agg      336
Ser Phe Arg Ser Ser Pro Trp Gln Gln Pro Ala Leu Leu Pro Gln Arg
            100                 105                 110
agg cga gga cac acg ccc gaa ggt gcg acg gcg ctt ccc ggc gct ccg      384
Arg Arg Gly His Thr Pro Glu Gly Ala Thr Ala Leu Pro Gly Ala Pro
        115                 120                 125
gct gcc aaa ggg gaa cca gag gag gag gat ggg ggc gcg gct gac cct      432
Ala Ala Lys Gly Glu Pro Glu Glu Glu Asp Gly Gly Ala Ala Asp Pro
    130                 135                 140
cgg aag ggt ggc cgg ccg ggg agc agc cac aac ggc agc ggg gac ggg      480
Arg Lys Gly Gly Arg Pro Gly Ser Ser His Asn Gly Ser Gly Asp Gly
145                 150                 155                 160
ggt gcc gct gtc ccg acc tcc gga ccc ggg gac ttc ctg tac gtg ggt      528
Gly Ala Ala Val Pro Thr Ser Gly Pro Gly Asp Phe Leu Tyr Val Gly
                165                 170                 175

gtg atg acc gca cag aag tac ctg ggc agt cgc gcg ctg gcc gcg cag 576
Val Met Thr Ala Gln Lys Tyr Leu Gly Ser Arg Ala Leu Ala Ala Gln
            180                 185                 190
cgg acc tgg gcg cgc ttc atc cct ggc cgc gtg gag ttc ttt tcc agt 624
Arg Thr Trp Ala Arg Phe Ile Pro Gly Arg Val Glu Phe Phe Ser Ser
        195                 200                 205
cag caa tct ccc agt gct gcg ctt ggc cag ccc ccg cca cct ttg cct 672
Gln Gln Ser Pro Ser Ala Ala Leu Gly Gln Pro Pro Pro Pro Leu Pro
    210                 215                 220
gtc atc gcg ctg cca ggg gtc gac gat tcc tac cct ccc cag aaa aag 720
Val Ile Ala Leu Pro Gly Val Asp Asp Ser Tyr Pro Pro Gln Lys Lys
225                 230                 235                 240
tcc ttc atg atg atc aag tac atg cac gac cac tat ctg gac aag tat 768
Ser Phe Met Met Ile Lys Tyr Met His Asp His Tyr Leu Asp Lys Tyr
                245                 250                 255
gag tgg ttc atg cgc gcc gac gac gat gtc tac atc aaa ggt gat aag 816
Glu Trp Phe Met Arg Ala Asp Asp Asp Val Tyr Ile Lys Gly Asp Lys
            260                 265                 270
tta gaa gaa ttt cta aga tcc cta aat agc agc aag cct ctc tac ctg 864
Leu Glu Glu Phe Leu Arg Ser Leu Asn Ser Ser Lys Pro Leu Tyr Leu
        275                 280                 285
gga cag acg ggc ctg ggc aat act gaa gaa ctt gga aag ctg ggg ctg 912
Gly Gln Thr Gly Leu Gly Asn Thr Glu Glu Leu Gly Lys Leu Gly Leu
    290                 295                 300
gag ccc ggg gag aac ttc tgc atg gga gga cct ggc atg atc ttc agc 960
Glu Pro Gly Glu Asn Phe Cys Met Gly Gly Pro Gly Met Ile Phe Ser
305                 310                 315                 320
aga gag gtt ctc agg cgg atg gtg cct cat atc ggc gaa tgc ctc cga 1008
Arg Glu Val Leu Arg Arg Met Val Pro His Ile Gly Glu Cys Leu Arg
                325                 330                 335
gag atg tac acc aca cac gaa gac gta gaa gta gga agg tgt gtt cgc 1056
Glu Met Tyr Thr Thr His Glu Asp Val Glu Val Gly Arg Cys Val Arg
            340                 345                 350
cgt ttc ggc ggg acg cag tgt gtc tgg tct tat gag atg cag cag ctg 1104
Arg Phe Gly Gly Thr Gln Cys Val Trp Ser Tyr Glu Met Gln Gln Leu
        355                 360                 365
ttc cat gaa aac tac gaa cac aat cgc aag ggt tac atc caa gac ctc 1152

Phe His Glu Asn Tyr Glu His Asn Arg Lys Gly Tyr Ile Gln Asp Leu
    370                 375                 380
cac aac agc aaa atc cac gca gcc atc acg ctc cat ccg aac aaa agg 1200
His Asn Ser Lys Ile His Ala Ala Ile Thr Leu His Pro Asn Lys Arg
385                 390                 395                 400
ccc gcg tac cag tac aga ctt cat aac tac atg ctc agc cgc aag atc 1248
Pro Ala Tyr Gln Tyr Arg Leu His Asn Tyr Met Leu Ser Arg Lys Ile
                405                 410                 415
tcc gag ctc cgc tac cgc acc atc cag ctc cac cgg gag agc gct ctc 1296
Ser Glu Leu Arg Tyr Arg Thr Ile Gln Leu His Arg Glu Ser Ala Leu
            420                 425                 430
atg agc aag ctc agc aac agt gaa gtg agc aaa gag gac caa cag ctg 1344
Met Ser Lys Leu Ser Asn Ser Glu Val Ser Lys Glu Asp Gln Gln Leu
        435                 440                 445
gga agg acg ccg tcc ttc aac cac ttc cag cct cgg gag aga aat gaa 1392
Gly Arg Thr Pro Ser Phe Asn His Phe Gln Pro Arg Glu Arg Asn Glu
    450                 455                 460
gtc atg gag tgg gag ttc ctg acg ggg aag ctg ctt tac tca gct gca 1440
Val Met Glu Trp Glu Phe Leu Thr Gly Lys Leu Leu Tyr Ser Ala Ala
465                 470                 475                 480
gag aac cag cct cct cga cag agc atc aac agc atc cta agg tca gct 1488
Glu Asn Gln Pro Pro Arg Gln Ser Ile Asn Ser Ile Leu Arg Ser Ala
                485                 490                 495
ctg gat gac act gtc ctg cag gtg atg gag atg atc aac gag aat gcc 1536
Leu Asp Asp Thr Val Leu Gln Val Met Glu Met Ile Asn Glu Asn Ala
            500                 505                 510
aag agt agg ggc cga ctc atc gac ttc aag gaa att cag tat ggc tac 1584
Lys Ser Arg Gly Arg Leu Ile Asp Phe Lys Glu Ile Gln Tyr Gly Tyr
        515                 520                 525
cgc agg gtt gat ccc atg cac ggg gtt gag tac ata ttg gac ctg cta 1632
Arg Arg Val Asp Pro Met His Gly Val Glu Tyr Ile Leu Asp Leu Leu
    530                 535                 540
ctc ctg tac aaa agg cac aaa gga agg aaa ctg act gtg cct gtg agg 1680
Leu Leu Tyr Lys Arg His Lys Gly Arg Lys Leu Thr Val Pro Val Arg
545                 550                 555                 560
cgc cat gcc tat ctt cag cag cca ttt agc aag cct ttc ttc aga gag 1728
Arg His Ala Tyr Leu Gln Gln Pro Phe Ser Lys Pro Phe Phe Arg Glu

                565                 570                 575
gtg gaa gaa ctc gac gtc aac cgt ctg gtg gag agt atc aac agc ggt 1776
Val Glu Glu Leu Asp Val Asn Arg Leu Val Glu Ser Ile Asn Ser Gly
            580                 585                 590
aca cag tca ttc tcc gtt ata tcc aat tct cta aaa att ctc tct tct 1824
Thr Gln Ser Phe Ser Val Ile Ser Asn Ser Leu Lys Ile Leu Ser Ser
        595                 600                 605
ctt caa gag gcc aaa gac ata gga ggg cac aat gaa aag aaa gta cac 1872
Leu Gln Glu Ala Lys Asp Ile Gly Gly His Asn Glu Lys Lys Val His
    610                 615                 620
att ctc gtt cca ctc gtt gga agg tac gac att ttc ttg aga ttc atg 1920
Ile Leu Val Pro Leu Val Gly Arg Tyr Asp Ile Phe Leu Arg Phe Met
625                 630                 635                 640
gaa aat ttt gaa agt acg tgt ctt atc cca aag caa aat gtc aag ctt 1968
Glu Asn Phe Glu Ser Thr Cys Leu Ile Pro Lys Gln Asn Val Lys Leu
                645                 650                 655
gtc atc atc ctt ttc agc agg gat gct ggc caa gag tcc atc aag cac 2016
Val Ile Ile Leu Phe Ser Arg Asp Ala Gly Gln Glu Ser Ile Lys His
            660                 665                 670
att gag ctg ata caa gaa tat cag agc agg tat ccc agt gca gaa atg 2064
Ile Glu Leu Ile Gln Glu Tyr Gln Ser Arg Tyr Pro Ser Ala Glu Met
        675                 680                 685
atg ctc att ccc atg aag gga gag ttt tcc aga ggt ctt ggt ctt gaa 2112
Met Leu Ile Pro Met Lys Gly Glu Phe Ser Arg Gly Leu Gly Leu Glu
    690                 695                 700
atg gct tct tcc cag ttt gac aat gac aca ttg ctg cta ttt tgt gat 2160
Met Ala Ser Ser Gln Phe Asp Asn Asp Thr Leu Leu Leu Phe Cys Asp
705                 710                 715                 720
gtt gac ttg att ttc aga gga gac ttc ctc caa cgc tgt cga gac aat 2208
Val Asp Leu Ile Phe Arg Gly Asp Phe Leu Gln Arg Cys Arg Asp Asn
                725                 730                 735
aca gtt cag gga caa cag gta tat tac ccc atc atc ttt agc cag tat 2256
Thr Val Gln Gly Gln Gln Val Tyr Tyr Pro Ile Ile Phe Ser Gln Tyr
            740                 745                 750
gac cca aag gtc acc cat atg aga aat cct ccc aca gag ggt gac ttt 2304
Asp Pro Lys Val Thr His Met Arg Asn Pro Pro Thr Glu Gly Asp Phe
        755                 760                 765

gta ttc tca aag gaa act ggg ttt tgg aga gac tat ggc tac gga atc 2352
Val Phe Ser Lys Glu Thr Gly Phe Trp Arg Asp Tyr Gly Tyr Gly Ile
    770                 775                 780
aca tgc att tac aaa agc gat cta ctg ggt gca ggt gga ttt gat acc 2400
Thr Cys Ile Tyr Lys Ser Asp Leu Leu Gly Ala Gly Gly Phe Asp Thr
785                 790                 795                 800
tca ata caa ggc tgg gga ctg gaa gat gta gat ctc tat aat aaa gtc 2448
Ser Ile Gln Gly Trp Gly Leu Glu Asp Val Asp Leu Tyr Asn Lys Val
                805                 810                 815
atc cta tct ggc tta cgg ccc ttc aga agt caa gaa gtg gga gtg gtg 2496
Ile Leu Ser Gly Leu Arg Pro Phe Arg Ser Gln Glu Val Gly Val Val
            820                 825                 830
cat att ttc cat cct gtt cat tgt gat cct aac ttg gac cct aag cag 2544
His Ile Phe His Pro Val His Cys Asp Pro Asn Leu Asp Pro Lys Gln
        835                 840                 845
tat aag atg tgc tta gga tcc aaa gca agt act ttt gcc tca acc atg 2592
Tyr Lys Met Cys Leu Gly Ser Lys Ala Ser Thr Phe Ala Ser Thr Met
    850                 855                 860
caa ctg gct gaa ctc tgg tta gaa aaa cat ttg ggt gtc agg gat aat 2640
Gln Leu Ala Glu Leu Trp Leu Glu Lys His Leu Gly Val Arg Asp Asn
865                 870                 875                 880
cga act ctc tcc 2652
Arg Thr Leu Ser

<210> 66
<211> 884
<212> PRT
<213> mouse

<400> 66

Met Ala Val Arg Ser Arg Arg Pro Trp Val Ser Val Ala Leu Gly Leu
1               5                   10                  15

Val Leu Gly Phe Thr Ala Ala Ser Trp Leu Ile Ala Pro Arg Val Ala
            20                  25                  30

Glu Leu Ser Glu Lys Arg Arg Arg Gly Ser Ser Leu Cys Ser Tyr Tyr
        35                  40                  45

Gly Arg Ser Ala Thr Gly Pro Arg Ala Asp Ala Gln Gln Leu Leu Pro
    50                  55                  60

Gln Pro Gln Ser Arg Pro Arg Leu Glu Gln Ser Pro Pro Pro Ala Ser
65                  70                  75                  80

His Glu Leu Pro Gly Pro Gln Gln Pro Glu Ala Ala Pro Gly Gly Pro
                85                  90                  95

Ser Phe Arg Ser Ser Pro Trp Gln Gln Pro Ala Leu Leu Pro Gln Arg
            100                 105                 110

Arg Arg Gly His Thr Pro Glu Gly Ala Thr Ala Leu Pro Gly Ala Pro
        115                 120                 125

Ala Ala Lys Gly Glu Pro Glu Glu Glu Asp Gly Gly Ala Ala Asp Pro
    130                 135                 140

Arg Lys Gly Gly Arg Pro Gly Ser Ser His Asn Gly Ser Gly Asp Gly
145                 150                 155                 160

Gly Ala Ala Val Pro Thr Ser Gly Pro Gly Asp Phe Leu Tyr Val Gly
                165                 170                 175

Val Met Thr Ala Gln Lys Tyr Leu Gly Ser Arg Ala Leu Ala Ala Gln
            180                 185                 190

Arg Thr Trp Ala Arg Phe Ile Pro Gly Arg Val Glu Phe Phe Ser Ser
        195                 200                 205

Gln Gln Ser Pro Ser Ala Ala Leu Gly Gln Pro Pro Pro Pro Leu Pro
    210                 215                 220

Val Ile Ala Leu Pro Gly Val Asp Asp Ser Tyr Pro Pro Gln Lys Lys
225                 230                 235                 240

Ser Phe Met Met Ile Lys Tyr Met His Asp His Tyr Leu Asp Lys Tyr
                245                 250                 255

Glu Trp Phe Met Arg Ala Asp Asp Asp Val Tyr Ile Lys Gly Asp Lys
            260                 265                 270

Leu Glu Glu Phe Leu Arg Ser Leu Asn Ser Ser Lys Pro Leu Tyr Leu
        275                 280                 285

Gly Gln Thr Gly Leu Gly Asn Thr Glu Glu Leu Gly Lys Leu Gly Leu
    290                 295                 300

Glu Pro Gly Glu Asn Phe Cys Met Gly Gly Pro Gly Met Ile Phe Ser
305                 310                 315                 320

Arg Glu Val Leu Arg Arg Met Val Pro His Ile Gly Glu Cys Leu Arg
                325                 330                 335

Glu Met Tyr Thr Thr His Glu Asp Val Glu Val Gly Arg Cys Val Arg
            340                 345                 350

Arg Phe Gly Gly Thr Gln Cys Val Trp Ser Tyr Glu Met Gln Gln Leu
        355                 360                 365

Phe His Glu Asn Tyr Glu His Asn Arg Lys Gly Tyr Ile Gln Asp Leu
    370                 375                 380

His Asn Ser Lys Ile His Ala Ala Ile Thr Leu His Pro Asn Lys Arg
385                 390                 395                 400

Pro Ala Tyr Gln Tyr Arg Leu His Asn Tyr Met Leu Ser Arg Lys Ile
                405                 410                 415

Ser Glu Leu Arg Tyr Arg Thr Ile Gln Leu His Arg Glu Ser Ala Leu
            420                 425                 430

Met Ser Lys Leu Ser Asn Ser Glu Val Ser Lys Glu Asp Gln Gln Leu
        435                 440                 445

Gly Arg Thr Pro Ser Phe Asn His Phe Gln Pro Arg Glu Arg Asn Glu
    450                 455                 460

Val Met Glu Trp Glu Phe Leu Thr Gly Lys Leu Leu Tyr Ser Ala Ala

465                 470                 475                 480

Glu Asn Gln Pro Pro Arg Gln Ser Ile Asn Ser Ile Leu Arg Ser Ala
                485                 490                 495

Leu Asp Asp Thr Val Leu Gln Val Met Glu Met Ile Asn Glu Asn Ala
            500                 505                 510

Lys Ser Arg Gly Arg Leu Ile Asp Phe Lys Glu Ile Gln Tyr Gly Tyr
        515                 520                 525

Arg Arg Val Asp Pro Met His Gly Val Glu Tyr Ile Leu Asp Leu Leu
    530                 535                 540

Leu Leu Tyr Lys Arg His Lys Gly Arg Lys Leu Thr Val Pro Val Arg
545                 550                 555                 560

Arg His Ala Tyr Leu Gln Gln Pro Phe Ser Lys Pro Phe Phe Arg Glu
                565                 570                 575

Val Glu Glu Leu Asp Val Asn Arg Leu Val Glu Ser Ile Asn Ser Gly
            580                 585                 590

Thr Gln Ser Phe Ser Val Ile Ser Asn Ser Leu Lys Ile Leu Ser Ser
        595                 600                 605

Leu Gln Glu Ala Lys Asp Ile Gly Gly His Asn Glu Lys Lys Val His
    610                 615                 620

Ile Leu Val Pro Leu Val Gly Arg Tyr Asp Ile Phe Leu Arg Phe Met
625                 630                 635                 640

Glu Asn Phe Glu Ser Thr Cys Leu Ile Pro Lys Gln Asn Val Lys Leu
                645                 650                 655

Val Ile Ile Leu Phe Ser Arg Asp Ala Gly Gln Glu Ser Ile Lys His
            660                 665                 670

Ile Glu Leu Ile Gln Glu Tyr Gln Ser Arg Tyr Pro Ser Ala Glu Met
        675                 680                 685

Met Leu Ile Pro Met Lys Gly Glu Phe Ser Arg Gly Leu Gly Leu Glu
    690                 695                 700

Met Ala Ser Ser Gln Phe Asp Asn Asp Thr Leu Leu Leu Phe Cys Asp
705                 710                 715                 720

Val Asp Leu Ile Phe Arg Gly Asp Phe Leu Gln Arg Cys Arg Asp Asn
                725                 730                 735

Thr Val Gln Gly Gln Gln Val Tyr Tyr Pro Ile Ile Phe Ser Gln Tyr
            740                 745                 750

Asp Pro Lys Val Thr His Met Arg Asn Pro Pro Thr Glu Gly Asp Phe
        755                 760                 765

Val Phe Ser Lys Glu Thr Gly Phe Trp Arg Asp Tyr Gly Tyr Gly Ile
    770                 775                 780

Thr Cys Ile Tyr Lys Ser Asp Leu Leu Gly Ala Gly Gly Phe Asp Thr
785                 790                 795                 800

Ser Ile Gln Gly Trp Gly Leu Glu Asp Val Asp Leu Tyr Asn Lys Val
                805                 810                 815

Ile Leu Ser Gly Leu Arg Pro Phe Arg Ser Gln Glu Val Gly Val Val
            820                 825                 830

His Ile Phe His Pro Val His Cys Asp Pro Asn Leu Asp Pro Lys Gln
        835                 840                 845

Tyr Lys Met Cys Leu Gly Ser Lys Ala Ser Thr Phe Ala Ser Thr Met
    850                 855                 860

Gln Leu Ala Glu Leu Trp Leu Glu Lys His Leu Gly Val Arg Asp Asn
865                 870                 875                 880

Arg Thr Leu Ser

<210>  67
<211>  2490
<212>  DNA
<213>  mouse

<220>
<221> CDS
<222> (1)..(2325)
<223>

<400> 67
atg cgg gcg tcg ctg ctg ctg tcc gtg ctg cgg ccc gcg ggg ccc gtg 48
Met Arg Ala Ser Leu Leu Leu Ser Val Leu Arg Pro Ala Gly Pro Val
1               5                   10                  15
gcc gtg ggc atc tct ctg ggc ttc acc ctg agc ctg ctc agc gtc acc 96
Ala Val Gly Ile Ser Leu Gly Phe Thr Leu Ser Leu Leu Ser Val Thr
            20                  25                  30
tgg gtg gag gag cct tgc gga ccc ggg ccg ccc caa ccc gga gac tct 144
Trp Val Glu Glu Pro Cys Gly Pro Gly Pro Pro Gln Pro Gly Asp Ser
        35                  40                  45
gag ctg ccg ccg cgc ggc aac acc aac gcg gcg cgc cgg ccc aac tcg 192
Glu Leu Pro Pro Arg Gly Asn Thr Asn Ala Ala Arg Arg Pro Asn Ser
    50                  55                  60
gtg cag ccc gga tcc gag cgc gag agg ccc ggg gcc ggc gca ggc acc 240
Val Gln Pro Gly Ser Glu Arg Glu Arg Pro Gly Ala Gly Ala Gly Thr
65                  70                  75                  80
ggt gag agc tgg gag cct cgt gtc ttg ccc tac cat ccc gcg cag cca 288
Gly Glu Ser Trp Glu Pro Arg Val Leu Pro Tyr His Pro Ala Gln Pro
                85                  90                  95
ggc cag gcc acc aag aag gcc gtc aga act cgg tat atc agc acg gag 336
Gly Gln Ala Thr Lys Lys Ala Val Arg Thr Arg Tyr Ile Ser Thr Glu
            100                 105                 110
ctg ggc atc agg cag aag ctt ctg gtg gca gtg ctg acc tca caa gcc 384
Leu Gly Ile Arg Gln Lys Leu Leu Val Ala Val Leu Thr Ser Gln Ala
        115                 120                 125
acg ttg cct aca ctg ggt gtg gct gta aac cga act ctg gga cac cga 432
Thr Leu Pro Thr Leu Gly Val Ala Val Asn Arg Thr Leu Gly His Arg
    130                 135                 140
ctg gag cat gta gtg ttc ctg acc ggt gcg agg ggc cgc cgg aca cct 480
Leu Glu His Val Val Phe Leu Thr Gly Ala Arg Gly Arg Arg Thr Pro
145                 150                 155                 160

tca ggc atg gcg gtg gtg gca ctg ggc gaa gag agg ccc atc gga cac 528
Ser Gly Met Ala Val Val Ala Leu Gly Glu Glu Arg Pro Ile Gly His
                165                 170                 175
ctg cac ctg gcg ctg cgc cac ctg ctg gag caa cac ggc gat gac ttt 576
Leu His Leu Ala Leu Arg His Leu Leu Glu Gln His Gly Asp Asp Phe
            180                 185                 190
gac tgg ttt ttc cta gtg cct gat gcc acc tat act gaa gcg cat gga 624
Asp Trp Phe Phe Leu Val Pro Asp Ala Thr Tyr Thr Glu Ala His Gly
        195                 200                 205
ctg gac cgc cta gct ggc cac ctc agc ctt gct tca gca acc cat ctc 672
Leu Asp Arg Leu Ala Gly His Leu Ser Leu Ala Ser Ala Thr His Leu
    210                 215                 220
tat ctt ggc cgg ccg cag gac ttc atc ggt gga gat act acc cca ggc 720
Tyr Leu Gly Arg Pro Gln Asp Phe Ile Gly Gly Asp Thr Thr Pro Gly
225                 230                 235                 240
cgc tac tgc cac ggg ggc ttt gga gtc ttg ctc tct cgc aca ctg cta 768
Arg Tyr Cys His Gly Gly Phe Gly Val Leu Leu Ser Arg Thr Leu Leu
                245                 250                 255
cag caa ctg cgc ccc cac ctg gaa agc tgc cgc aac gac atc gtc agt 816
Gln Gln Leu Arg Pro His Leu Glu Ser Cys Arg Asn Asp Ile Val Ser
            260                 265                 270
gct cgc ccg gat gag tgg ttg ggc cgc tgc atc ctt gat gcc aca ggc 864
Ala Arg Pro Asp Glu Trp Leu Gly Arg Cys Ile Leu Asp Ala Thr Gly
        275                 280                 285
gtg ggc tgt act ggt gac cac gag gga atg cac tac aac tac ctg gaa 912
Val Gly Cys Thr Gly Asp His Glu Gly Met His Tyr Asn Tyr Leu Glu
    290                 295                 300
ctg agc ccc ggg gag cct gta cag gag ggg gac cct cgt ttc cgc agc 960
Leu Ser Pro Gly Glu Pro Val Gln Glu Gly Asp Pro Arg Phe Arg Ser
305                 310                 315                 320
gcc ttg aca gcc cat ccc gtg cgt gac cct gtg cac atg tac cag ctg 1008
Ala Leu Thr Ala His Pro Val Arg Asp Pro Val His Met Tyr Gln Leu
                325                 330                 335
cac aaa gct ttt gcc cgc gct gag ctg gac cgc acg tac cag gag att 1056
His Lys Ala Phe Ala Arg Ala Glu Leu Asp Arg Thr Tyr Gln Glu Ile
            340                 345                 350
caa gaa ttg cag tgg gag atc cag aat acc agc cga ctg gct gct gat 1104

Gln Glu Leu Gln Trp Glu Ile Gln Asn Thr Ser Arg Leu Ala Ala Asp
        355                 360                 365
ggg gag aga gcc tct gcc tgg cca gtg ggc atc cca gca ccg tct cgc      1152
Gly Glu Arg Ala Ser Ala Trp Pro Val Gly Ile Pro Ala Pro Ser Arg
    370                 375                 380
cct gcc tca cgc ttt gag gtt ctg cgc tgg gac tac ttc aca gaa caa      1200
Pro Ala Ser Arg Phe Glu Val Leu Arg Trp Asp Tyr Phe Thr Glu Gln
385                 390                 395                 400
tac gcg ttc tcc tgc gcc gat ggc tct ccc cgc tgc ccg ttg cgt ggg      1248
Tyr Ala Phe Ser Cys Ala Asp Gly Ser Pro Arg Cys Pro Leu Arg Gly
                405                 410                 415
gcc gac cag gct gat gtg gct gac gtc ctg ggg aca gcc tta gag gag      1296
Ala Asp Gln Ala Asp Val Ala Asp Val Leu Gly Thr Ala Leu Glu Glu
            420                 425                 430
ctc aac cgc cgt tac cag cca gcg ctg cag ctc cag aag caa cag ctg      1344
Leu Asn Arg Arg Tyr Gln Pro Ala Leu Gln Leu Gln Lys Gln Gln Leu
        435                 440                 445
gtg aac ggc tac cgg cgt ttt gat cca gcc cga ggc atg gag tac aca      1392
Val Asn Gly Tyr Arg Arg Phe Asp Pro Ala Arg Gly Met Glu Tyr Thr
    450                 455                 460
cta gac ctg cag ctg gaa gcg ctg aca ccc cag ggt ggc cgc tgg ccc      1440
Leu Asp Leu Gln Leu Glu Ala Leu Thr Pro Gln Gly Gly Arg Trp Pro
465                 470                 475                 480
ctc acc cgc agg gtg cag ctc ctt cgg ccc ttg agc cga gtg gag atc      1488
Leu Thr Arg Arg Val Gln Leu Leu Arg Pro Leu Ser Arg Val Glu Ile
                485                 490                 495
ttg cct gta ccc tat gtc acc gag gct tct cgg ctc act gtg cta ctg      1536
Leu Pro Val Pro Tyr Val Thr Glu Ala Ser Arg Leu Thr Val Leu Leu
            500                 505                 510
ccg ctg gct gca gcg gaa cga gac ctg gct tct ggc ttc tta gaa gcc      1584
Pro Leu Ala Ala Ala Glu Arg Asp Leu Ala Ser Gly Phe Leu Glu Ala
        515                 520                 525
ttt gcc act gca gcc ctg gaa cct ggt gat gca gca gcc ttg acc ctg      1632
Phe Ala Thr Ala Ala Leu Glu Pro Gly Asp Ala Ala Ala Leu Thr Leu
    530                 535                 540
ctg ctg ctg tat gag cca cgc cag gcc cag cgg gca gcc cac tca gac      1680
Leu Leu Leu Tyr Glu Pro Arg Gln Ala Gln Arg Ala Ala His Ser Asp

545                 550                 555                 560
gtc ttc gca cct gtc aag gcc cac gtg gca gag cta gag cgg cgt ttc     1728
Val Phe Ala Pro Val Lys Ala His Val Ala Glu Leu Glu Arg Arg Phe
                565                 570                 575
cct ggt gcc cgg gtg ccc tgg ctc agt gtg cag aca gca gcg ccc tct     1776
Pro Gly Ala Arg Val Pro Trp Leu Ser Val Gln Thr Ala Ala Pro Ser
            580                 585                 590
cca ctg cgt ctc atg gat ctg ctg tcc aag aag cac cca cta gac act     1824
Pro Leu Arg Leu Met Asp Leu Leu Ser Lys Lys His Pro Leu Asp Thr
        595                 600                 605
ctg ttc ctg ctg gcc ggg cca gac acg gta ctc aca cct gat ttc ctg     1872
Leu Phe Leu Leu Ala Gly Pro Asp Thr Val Leu Thr Pro Asp Phe Leu
    610                 615                 620
aac cgc tgc cgc atg cat gcc atc tct ggc tgg cag gcc ttc ttc ccc     1920
Asn Arg Cys Arg Met His Ala Ile Ser Gly Trp Gln Ala Phe Phe Pro
625                 630                 635                 640
atg cac ttc cag gcc ttc cac cct gct gtg gct cct cct cag ggc cct     1968
Met His Phe Gln Ala Phe His Pro Ala Val Ala Pro Pro Gln Gly Pro
                645                 650                 655
ggg cca cca gag ctg ggc cgt gac acc ggt cac ttt gat cgc cag gct     2016
Gly Pro Pro Glu Leu Gly Arg Asp Thr Gly His Phe Asp Arg Gln Ala
            660                 665                 670
gcc agt gag gca tgc ttc tac aac tcc gac tat gtg gcg gcc cgt ggc     2064
Ala Ser Glu Ala Cys Phe Tyr Asn Ser Asp Tyr Val Ala Ala Arg Gly
        675                 680                 685
cgg ctg gtg gcg gcc tcg gag cag gag gag gag ctg ctg gag agc ctg     2112
Arg Leu Val Ala Ala Ser Glu Gln Glu Glu Glu Leu Leu Glu Ser Leu
    690                 695                 700
gat gtg tac gag ttg ttt ctg cgc ttc tcc aac ttg cac gtg ctg aga     2160
Asp Val Tyr Glu Leu Phe Leu Arg Phe Ser Asn Leu His Val Leu Arg
705                 710                 715                 720
gca gta gag cca gcc ttg ctg cag cgc tac cgg gcc cag ccg tgc agt     2208
Ala Val Glu Pro Ala Leu Leu Gln Arg Tyr Arg Ala Gln Pro Cys Ser
                725                 730                 735
gca cgg ctc agt gaa gac ctt tac cac cgc tgt cgc cag agc gta ctt     2256
Ala Arg Leu Ser Glu Asp Leu Tyr His Arg Cys Arg Gln Ser Val Leu
            740                 745                 750

gag ggc ctt ggc tcc cgc acc cag ctt gcc atg ctg ctc ttt gag cag     2304
Glu Gly Leu Gly Ser Arg Thr Gln Leu Ala Met Leu Leu Phe Glu Gln
        755                 760                 765
gaa cag ggg aac agc acc taa gcccctgcac ctgtccctgc tcttccccag     2355
Glu Gln Gly Asn Ser Thr
    770
gaacctggag ccacgtgcca gcctcgctgg acagggctgg ctgtagcctc agtccctagg     2415
gcagcccact ggtcccttgt ctcttgcttt gttggaccca tgggctcagg acaagccctg     2475
agacagatgc cctag                                                      2490

<210>  68
<211>  774
<212>  PRT
<213>  mouse

<400>  68

Met Arg Ala Ser Leu Leu Leu Ser Val Leu Arg Pro Ala Gly Pro Val
1               5                   10                  15

Ala Val Gly Ile Ser Leu Gly Phe Thr Leu Ser Leu Leu Ser Val Thr
            20                  25                  30

Trp Val Glu Glu Pro Cys Gly Pro Gly Pro Pro Gln Pro Gly Asp Ser
        35                  40                  45

Glu Leu Pro Pro Arg Gly Asn Thr Asn Ala Ala Arg Arg Pro Asn Ser
    50                  55                  60

Val Gln Pro Gly Ser Glu Arg Glu Arg Pro Gly Ala Gly Ala Gly Thr
65                  70                  75                  80

Gly Glu Ser Trp Glu Pro Arg Val Leu Pro Tyr His Pro Ala Gln Pro
                85                  90                  95

Gly Gln Ala Thr Lys Lys Ala Val Arg Thr Arg Tyr Ile Ser Thr Glu
            100                 105                 110

Leu Gly Ile Arg Gln Lys Leu Leu Val Ala Val Leu Thr Ser Gln Ala
        115                 120                 125

Thr Leu Pro Thr Leu Gly Val Ala Val Asn Arg Thr Leu Gly His Arg
    130                 135                 140

Leu Glu His Val Val Phe Leu Thr Gly Ala Arg Gly Arg Arg Thr Pro
145                 150                 155                 160

Ser Gly Met Ala Val Val Ala Leu Gly Glu Glu Arg Pro Ile Gly His
                165                 170                 175

Leu His Leu Ala Leu Arg His Leu Leu Glu Gln His Gly Asp Asp Phe
            180                 185                 190

Asp Trp Phe Phe Leu Val Pro Asp Ala Thr Tyr Thr Glu Ala His Gly
        195                 200                 205

Leu Asp Arg Leu Ala Gly His Leu Ser Leu Ala Ser Ala Thr His Leu
    210                 215                 220

Tyr Leu Gly Arg Pro Gln Asp Phe Ile Gly Gly Asp Thr Thr Pro Gly

225                 230                 235                 240

Arg Tyr Cys His Gly Gly Phe Gly Val Leu Leu Ser Arg Thr Leu Leu
                245                 250                 255

Gln Gln Leu Arg Pro His Leu Glu Ser Cys Arg Asn Asp Ile Val Ser
            260                 265                 270

Ala Arg Pro Asp Glu Trp Leu Gly Arg Cys Ile Leu Asp Ala Thr Gly
        275                 280                 285

Val Gly Cys Thr Gly Asp His Glu Gly Met His Tyr Asn Tyr Leu Glu
    290                 295                 300

Leu Ser Pro Gly Glu Pro Val Gln Glu Gly Asp Pro Arg Phe Arg Ser
305                 310                 315                 320

Ala Leu Thr Ala His Pro Val Arg Asp Pro Val His Met Tyr Gln Leu
                325                 330                 335

His Lys Ala Phe Ala Arg Ala Glu Leu Asp Arg Thr Tyr Gln Glu Ile
            340                 345                 350

Gln Glu Leu Gln Trp Glu Ile Gln Asn Thr Ser Arg Leu Ala Ala Asp
        355                 360                 365

Gly Glu Arg Ala Ser Ala Trp Pro Val Gly Ile Pro Ala Pro Ser Arg
    370                 375                 380

Pro Ala Ser Arg Phe Glu Val Leu Arg Trp Asp Tyr Phe Thr Glu Gln
385                 390                 395                 400

Tyr Ala Phe Ser Cys Ala Asp Gly Ser Pro Arg Cys Pro Leu Arg Gly
                405                 410                 415

Ala Asp Gln Ala Asp Val Ala Asp Val Leu Gly Thr Ala Leu Glu Glu
            420                 425                 430

Leu Asn Arg Arg Tyr Gln Pro Ala Leu Gln Leu Gln Lys Gln Gln Leu
        435                 440                 445

Val Asn Gly Tyr Arg Arg Phe Asp Pro Ala Arg Gly Met Glu Tyr Thr
    450                 455                 460

Leu Asp Leu Gln Leu Glu Ala Leu Thr Pro Gln Gly Gly Arg Trp Pro
465                 470                 475                 480

Leu Thr Arg Arg Val Gln Leu Leu Arg Pro Leu Ser Arg Val Glu Ile
                485                 490                 495

Leu Pro Val Pro Tyr Val Thr Glu Ala Ser Arg Leu Thr Val Leu Leu
            500                 505                 510

Pro Leu Ala Ala Ala Glu Arg Asp Leu Ala Ser Gly Phe Leu Glu Ala
        515                 520                 525

Phe Ala Thr Ala Ala Leu Glu Pro Gly Asp Ala Ala Ala Leu Thr Leu
    530                 535                 540

Leu Leu Leu Tyr Glu Pro Arg Gln Ala Gln Arg Ala Ala His Ser Asp
545                 550                 555                 560

Val Phe Ala Pro Val Lys Ala His Val Ala Glu Leu Glu Arg Arg Phe
                565                 570                 575

Pro Gly Ala Arg Val Pro Trp Leu Ser Val Gln Thr Ala Ala Pro Ser
            580                 585                 590

Pro Leu Arg Leu Met Asp Leu Leu Ser Lys Lys His Pro Leu Asp Thr
        595                 600                 605

Leu Phe Leu Leu Ala Gly Pro Asp Thr Val Leu Thr Pro Asp Phe Leu
    610                 615                 620

Asn Arg Cys Arg Met His Ala Ile Ser Gly Trp Gln Ala Phe Phe Pro
625                 630                 635                 640

Met His Phe Gln Ala Phe His Pro Ala Val Ala Pro Pro Gln Gly Pro
                645                 650                 655

Gly Pro Pro Glu Leu Gly Arg Asp Thr Gly His Phe Asp Arg Gln Ala
            660                 665                 670

Ala Ser Glu Ala Cys Phe Tyr Asn Ser Asp Tyr Val Ala Ala Arg Gly
        675                 680                 685

Arg Leu Val Ala Ala Ser Glu Gln Glu Glu Glu Leu Leu Glu Ser Leu
    690                 695                 700

Asp Val Tyr Glu Leu Phe Leu Arg Phe Ser Asn Leu His Val Leu Arg
705                 710                 715                 720

Ala Val Glu Pro Ala Leu Leu Gln Arg Tyr Arg Ala Gln Pro Cys Ser
                725                 730                 735

Ala Arg Leu Ser Glu Asp Leu Tyr His Arg Cys Arg Gln Ser Val Leu
            740                 745                 750

Glu Gly Leu Gly Ser Arg Thr Gln Leu Ala Met Leu Leu Phe Glu Gln
        755                 760                 765

Glu Gln Gly Asn Ser Thr
    770

<210> 69
<211> 984
<212> DNA
<213> mouse

<220>
<221> CDS
<222> (1)..(984)
<223>

<400> 69
atg ttg ccc tcc cgg agg aaa gcg gcg cag ctg ccc tgg gag gat ggc 48
Met Leu Pro Ser Arg Arg Lys Ala Ala Gln Leu Pro Trp Glu Asp Gly
1               5                   10                  15
agg gcc agg ttg ctt cct gga ggc ctc cgc cgg aaa tgc tcc atc ttc 96
Arg Ala Arg Leu Leu Pro Gly Gly Leu Arg Arg Lys Cys Ser Ile Phe
            20                  25                  30
cac ctc ttc att gcc ttt ctc ctg ttg gtc ttc ttc tcc ctg ctc tgg 144
His Leu Phe Ile Ala Phe Leu Leu Leu Val Phe Phe Ser Leu Leu Trp
        35                  40                  45
ctg cag ctc agc tgt tct gga gat atg gcc cag gtg acc agg gga caa 192
Leu Gln Leu Ser Cys Ser Gly Asp Met Ala Gln Val Thr Arg Gly Gln
    50                  55                  60
ggg caa gag acc tcg ggt cca ccc cgg gct tgc cct cca gag ccg ccc 240
Gly Gln Glu Thr Ser Gly Pro Pro Arg Ala Cys Pro Pro Glu Pro Pro
65                  70                  75                  80
cct gag cac tgg gaa gaa gat gag tcc tgg ggc ccc cac cgc ttg gca 288
Pro Glu His Trp Glu Glu Asp Glu Ser Trp Gly Pro His Arg Leu Ala
                85                  90                  95
gtg ctg gtg ccc ttt cgt gag cgc ttt gag gag ctg ctg gtc ttt gtg 336
Val Leu Val Pro Phe Arg Glu Arg Phe Glu Glu Leu Leu Val Phe Val
            100                 105                 110
ccc cac atg cac cgc ttc cta agc agg aag agg atc cag cac cac atc 384
Pro His Met His Arg Phe Leu Ser Arg Lys Arg Ile Gln His His Ile
        115                 120                 125
tat gtg ctc aac cag gtg gac cat ttc agg ttc aat cgg gca gca ctc 432
Tyr Val Leu Asn Gln Val Asp His Phe Arg Phe Asn Arg Ala Ala Leu
    130                 135                 140
atc aat gtg ggc ttc ctg gag agc agc aac agc aca gac tac atc gcc 480
Ile Asn Val Gly Phe Leu Glu Ser Ser Asn Ser Thr Asp Tyr Ile Ala
145                 150                 155                 160
atg cac gat gtg gac ctg ctc cct ctc aat gag gag ctg gac tat ggc 528
Met His Asp Val Asp Leu Leu Pro Leu Asn Glu Glu Leu Asp Tyr Gly
                165                 170                 175
ttc ccg gag gct ggg ccc ttc cat gtg gcc tcc cca gag ctc cac cct 576
Phe Pro Glu Ala Gly Pro Phe His Val Ala Ser Pro Glu Leu His Pro

180                 185                 190
ctc tac cac tac aag acc tat gtg ggc ggc att ctg ctg ctg tcc aaa     624
Leu Tyr His Tyr Lys Thr Tyr Val Gly Gly Ile Leu Leu Leu Ser Lys
        195                 200                 205
cag cac tac cag ctg tgc aac gga atg tcc aac cgc ttt tgg ggc tgg     672
Gln His Tyr Gln Leu Cys Asn Gly Met Ser Asn Arg Phe Trp Gly Trp
    210                 215                 220
ggc cga gag gat gat gaa ttc tac cgg cgc atc aaa gga gct ggc ctc     720
Gly Arg Glu Asp Asp Glu Phe Tyr Arg Arg Ile Lys Gly Ala Gly Leu
225                 230                 235                 240
cag ctt ttc cgc ccc tcg gga atc aca act ggg tac cag aca ttt cgc     768
Gln Leu Phe Arg Pro Ser Gly Ile Thr Thr Gly Tyr Gln Thr Phe Arg
                245                 250                 255
cac ttg cat gac cct gcc tgg cgg aag agg gac caa aaa cgc att gcg     816
His Leu His Asp Pro Ala Trp Arg Lys Arg Asp Gln Lys Arg Ile Ala
            260                 265                 270
gct caa aaa cag gaa cag ttc aag gtg gac cgg gag gga ggc ctg aac     864
Ala Gln Lys Gln Glu Gln Phe Lys Val Asp Arg Glu Gly Gly Leu Asn
        275                 280                 285
act gtg aag tac cgg gtg gat tcc cgc acg gca ctg tct ata gga ggg     912
Thr Val Lys Tyr Arg Val Asp Ser Arg Thr Ala Leu Ser Ile Gly Gly
    290                 295                 300
gcc ccg tgc act gtc ctc aat gtc atg ctg gac tgc gat aaa aca gcc     960
Ala Pro Cys Thr Val Leu Asn Val Met Leu Asp Cys Asp Lys Thr Ala
305                 310                 315                 320
acc cca tgg tgc ata ttt ggc tga                                     984
Thr Pro Trp Cys Ile Phe Gly
                325

<210> 70
<211> 327
<212> PRT
<213> mouse

<400> 70

Met Leu Pro Ser Arg Arg Lys Ala Ala Gln Leu Pro Trp Glu Asp Gly

1                   5                   10                  15

Arg Ala Arg Leu Leu Pro Gly Gly Leu Arg Arg Lys Cys Ser Ile Phe
                20                  25                  30

His Leu Phe Ile Ala Phe Leu Leu Leu Val Phe Phe Ser Leu Leu Trp
            35                  40                  45

Leu Gln Leu Ser Cys Ser Gly Asp Met Ala Gln Val Thr Arg Gly Gln
    50                  55                  60

Gly Gln Glu Thr Ser Gly Pro Pro Arg Ala Cys Pro Pro Glu Pro Pro
65                  70                  75                  80

Pro Glu His Trp Glu Glu Asp Glu Ser Trp Gly Pro His Arg Leu Ala
                85                  90                  95

Val Leu Val Pro Phe Arg Glu Arg Phe Glu Glu Leu Leu Val Phe Val
                100                 105                 110

Pro His Met His Arg Phe Leu Ser Arg Lys Arg Ile Gln His His Ile
            115                 120                 125

Tyr Val Leu Asn Gln Val Asp His Phe Arg Phe Asn Arg Ala Ala Leu
    130                 135                 140

Ile Asn Val Gly Phe Leu Glu Ser Ser Asn Ser Thr Asp Tyr Ile Ala
145                 150                 155                 160

Met His Asp Val Asp Leu Leu Pro Leu Asn Glu Glu Leu Asp Tyr Gly
                165                 170                 175

Phe Pro Glu Ala Gly Pro Phe His Val Ala Ser Pro Glu Leu His Pro
            180                 185                 190

Leu Tyr His Tyr Lys Thr Tyr Val Gly Gly Ile Leu Leu Leu Ser Lys
        195                 200                 205

Gln His Tyr Gln Leu Cys Asn Gly Met Ser Asn Arg Phe Trp Gly Trp
    210                 215                 220

Gly Arg Glu Asp Asp Glu Phe Tyr Arg Arg Ile Lys Gly Ala Gly Leu
225                 230                 235                 240

Gln Leu Phe Arg Pro Ser Gly Ile Thr Thr Gly Tyr Gln Thr Phe Arg
                245                 250                 255

His Leu His Asp Pro Ala Trp Arg Lys Arg Asp Gln Lys Arg Ile Ala
            260                 265                 270

Ala Gln Lys Gln Glu Gln Phe Lys Val Asp Arg Glu Gly Gly Leu Asn
        275                 280                 285

Thr Val Lys Tyr Arg Val Asp Ser Arg Thr Ala Leu Ser Ile Gly Gly
    290                 295                 300

Ala Pro Cys Thr Val Leu Asn Val Met Leu Asp Cys Asp Lys Thr Ala
305                 310                 315                 320

Thr Pro Trp Cys Ile Phe Gly
                325

## Description

### TECHNICAL FIELD

This invention relates to a method of detecting Paget disease of bone and, more specifically, to a method of detecting Paget disease of bone by associating Paget disease of bone with the mutation in a gene coding for an enzyme that takes part in the synthesis of chondroitin/chondroitin sulfate or the amount of expression of said gene.

### BACKGROUND ART

Paget disease of bone is a disease characterized by concomitant increase of osteoclasts and osteoblasts which leads to localized abnormal enhancement of bone resorption and the accompanying secondary bone formation, eventually causing bone deformation and thickening. It is held predominantly that the onset of Paget disease of bone is triggered by a viral infection and Reference 1 states that measles virus could be detected by reverse transcription-polymerase chain reaction method (RT-PCR method) using RNA extracted from peripheral blood- and bone marrow-derived mononuclear cells from patients. However, the reference does not show that measles virus is a direct cause of Paget disease of bone and the pathological etiology for Paget disease of bone is unknown. Patients with this disease are rare in Japan but a lot of patients are found in Europe and the United States of America and according to Reference 2, the incidence of the disease increases with age and is higher in men than in women.

Mutant gene loci that are responsible for Paget disease of bone are already known and described in Reference 3; however, more than one gene is present on those loci and it has not been known which gene should mutate to induce Paget disease of bone. Therefore, in order to diagnose Paget disease of bone by a convenient method, it has been necessary to identify which gene should mutate for the disease to manifest itself.

### DISCLOSURE OF THE INVENTION

There has been a growing expectation for scientists to identify the causative gene responsible for the pathology of Paget disease of bone and to develop a convenient method for detecting Paget disease of bone.

### BRIEF DESCRIPTION OF THE DRAWINGS

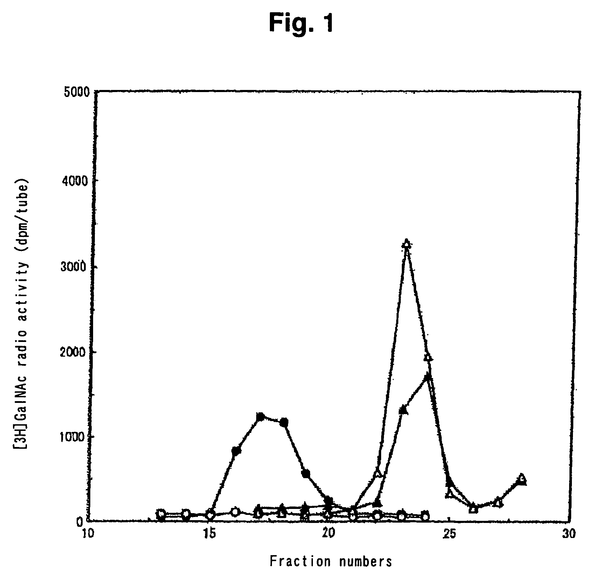

Fig. 1 shows a chromatographic chart for the synthesis of odd-numbered saccharides by the GalNAc transfer activity of K3; open circles show the activity for transferring GalNAc to chondroitin sulfate; solid circles show the activity for transferring GalNAc to chondroitin; open triangles show the activity for transferring GalNAc to chondroitin sulfate decasaccharide; and solid triangles show the activity for transferring GalNAc to chondroitin decasaccharide.

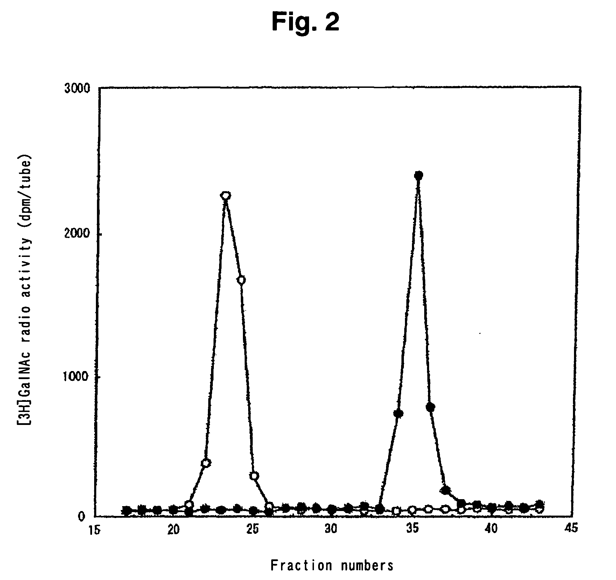

Fig. 2 shows a chromatographic chart for the undecasaccharide prepared by the GalNAc transfer activity of K3 and the product of its digestion with chondroitinase ACII; open circles refer to the undecasaccharide not digested with chondroitinase ACII; and solid circles refer to the product of digestion with chondroitinase ACII.

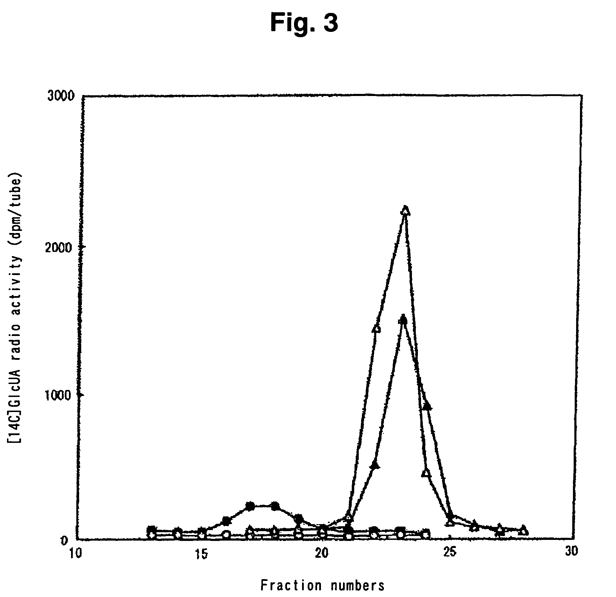

Fig. 3 shows a chromatographic chart for the synthesis of even-numbered saccharides by the GlcUA transfer activity of K3; open circles show the activity for transferring GlcUA to chondroitin sulfate; solid circles show the activity for transferring GlcUA to chondroitin; open triangles show the activity for transferring GlcUA to chondroitin sulfate undecasaccharide; and solid triangles show the activity for transferring GlcUA to chondroitin undecasaccharide.

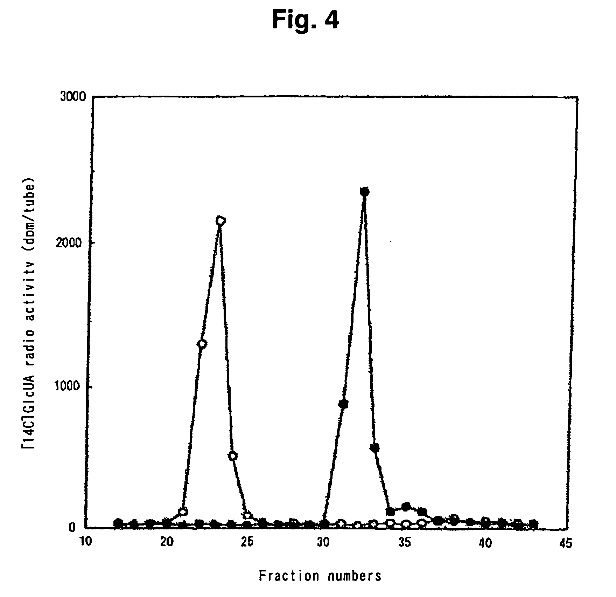

Fig. 4 shows a chromatographic chart for the dodecasaccharide prepared by the GlcUA transfer activity of K3 and the product of its digestion with chondroitinase ACII; open circles refer to the dodecasaccharide not digested with chondroitinase ACII; and solid circles refer to the product of digestion with chondroitinase ACII.

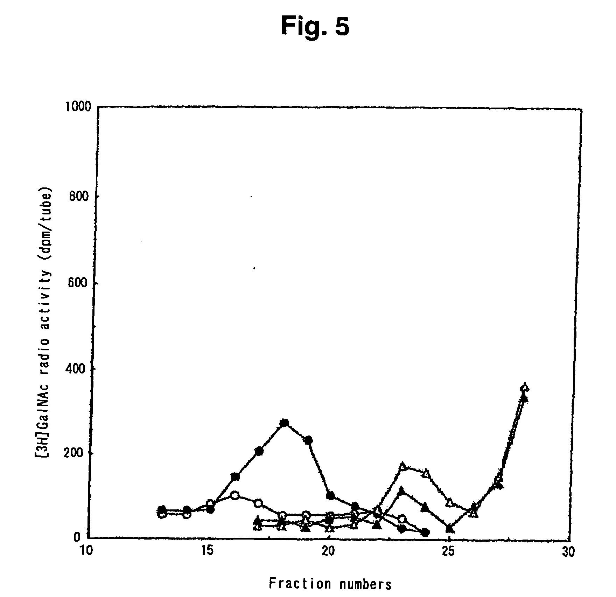

Fig. 5 shows a chromatographic chart for the synthesis of odd-numbered saccharides by the GalNAc transfer activity of K11; open circles show the activity for transferring GalNAc to chondroitin sulfate; solid circles show the activity for transferring GalNAc to chondroitin; open triangles show the activity for transferring GalNAc to chondroitin sulfate decasaccharide; and solid triangles show the activity for transferring GalNAc to chondroitin decasaccharide.

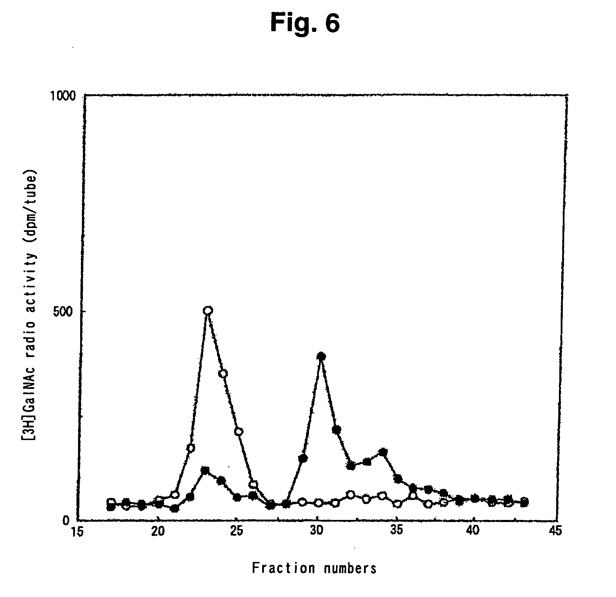

Fig. 6 shows a chromatographic chart for the undecasaccharide prepared by the GalNAc transfer activity of K11 and the product of its digestion with chondroitinase ACII; open circles refer to the undecasaccharide not digested with chondroitinase ACII; and solid circles refer to the product of digestion with chondroitinase ACII.

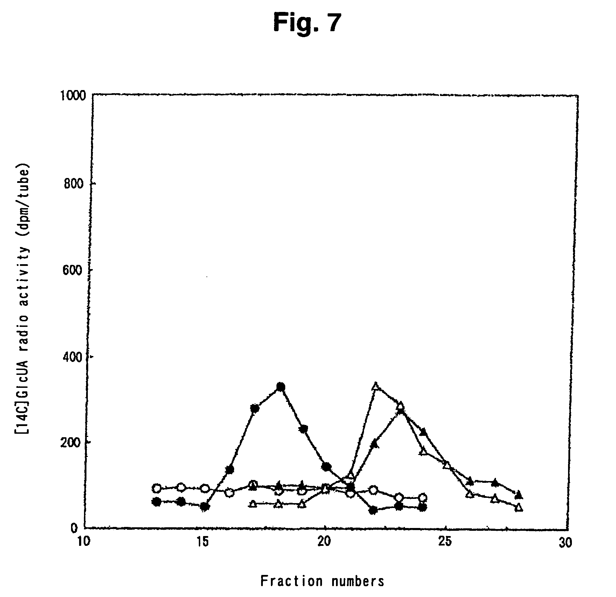

Fig. 7 shows a chromatographic chart for the synthesis of even-numbered saccharides by the GlcUA transfer activity of K11; open circles show the activity for transferring GlcUA to chondroitin sulfate; solid circles show the activity for transferring GlcUA to chondroitin; open triangles show the activity for transferring GlcUA to chondroitin sulfate undecasaccharide; and solid triangles show the activity for transferring GlcUA to chondroitin undecasaccharide.

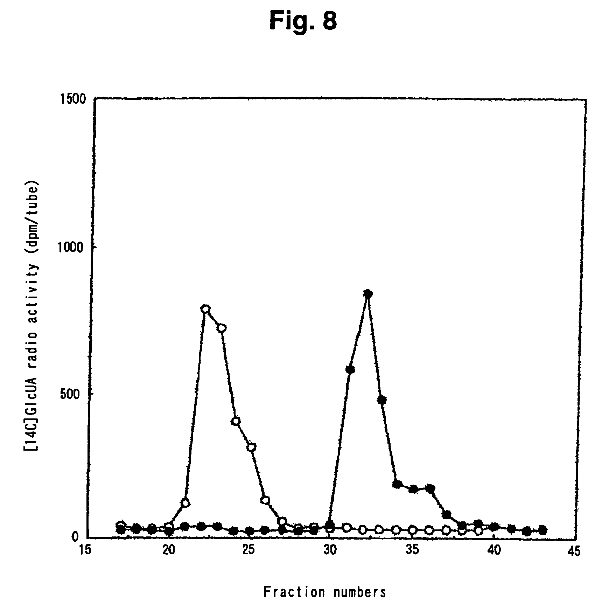

Fig. 8 shows a chromatographic chart for the dodecasaccharide prepared by the GlcUA transfer activity of K11 and the product of its digestion with chondroitinase ACII; open circles refer to the dodecasaccharide not digested with chondroitinase ACII; and solid circles refer to the product of digestion with chondroitinase ACII.

### DETAILED DESCRIPTION OF THE INVENTION

The present inventors made intensive studies with a view to solving the above-described problem and found that each of the gene mutations leading to Paget disease of bone occurred in the locus of DNA that would take part in the synthesis of chondroitin/chondroitin sulfate; the inventors applied this finding to the detection of Paget disease of bone and eventually completed the present invention.

The present invention is summarized as a method of detecting Paget disease of bone by associating the disease with the mutation in a gene coding a chondroitin synthase or the amount of expression of said gene.

### MODES FOR CARRYING OUT THE INVENTION

On the pages that follow, the present invention is described in detail by reference to various modes for carrying out it.

The detection method of the present invention is one for detecting Paget disease of bone by associating Paget disease of bone with the mutation of a gene coding a chondroitin synthase or the amount of expression of said gene.

The "chondroitin synthase" that can be used in the detection method of the present invention is not limited in any particular way as long as it is an enzyme that takes part in the synthesis of chondroitin or chondroitin sulfate. To be more specific, chondroitin and chondroitin sulfate are usually synthesized in vivo as proteoglycans, so not only enzymes that take part in extending the building blocks of chondroitin/chondroitin sulfate but also other enzymes such as those that synthesize the linkage between the protein of proteoglycan and glycosaminoglycan are encompassed. If these enzymes mutate or if they are not expressed, there will be no synthesis of chondroitin/chondroitin sulfate.

The "chondroitin synthase" that can be used in such detection method of the present invention is preferably a glycosyltransferase having activity for transferring a D-glucuronic acid residue or an N-acetyl-D-galactosamine residue to the saccharide residue at the non-reducing terminal of chondroitin/chondroitin sulfate (which may hereunder be also designated "glycosyltransferase 1" for convenience sake) or a glycosyltransferase by means of which a xylose residue linked to an amino acid residue has D-galactose linked thereto by a β1,4-glycoside linkage (which may hereunder be also designated "glycosyltransferase 2" for convenience sake).

The "glycosyltransferase 1" mentioned above is not limited in any particular way as long as it has activity for transferring a D-glucuronic acid (hereunder also referred to simply as GlcUA) residue or an N-acetyl-D-galactosamine (hereunder also referred to simply as N-acetylgalactosamine or GalNAc) residue to the saccharide residue at the non-reducing terminal of chondroitin/chondroitin sulfate. Such glycosyltransferase 1 takes part in extending chondroitin/chondroitin sulfate and if it mutates or is suppressed in expression, normal synthesis of chondroitin/chondroitin sulfate is believed to be prevented, leading to Paget disease of bone which can cause abnormalities in bone formation.

"Chondroitin" as it appears in the above definition of "glycosyltransferase 1" is a kind of glycosaminoglycan having the building blocks in which disaccharides each composed of a uronic acid (hereunder also referred to simply as "UA") residue and a GalNAc residue are linked by a 1,3 glycoside linkage are repeatedly linked by a β1,4 glycoside linkage and which are represented by -[4UAl-3GalNAcβ1-]ₙ (n is an integer of 2 or more). Chondroitin has no sulfuric acid group linked to these building blocks but chondroitin sulfate has the carbon atom sulfated at 2-, 4- or 6-position of the GalNAc residue or at 2-position of the uronic acid residue.

The "saccharide residue at the non-reducing terminal of chondroitin/chondroitin sulfate" is any one of the GalNAc residue and the UA residue that make up the building blocks of chondroitin/chondroitin sulfate, and "glycosyltransferase 1" is an enzyme that transfers UA when the saccharide residue under consideration is the GalNAc residue and transfers GalNAc when it is the UA residue but which at least has the activity for transferring either UA or GalNAc.

Specific examples of such "glycosyltransferase 1" include a chondroitin synthase containing a protein comprising the amino acid sequence of amino acid Nos. 1-882 depicted as SEQ ID NO:2.

Said enzyme has the activity for transferring the GalNAc residue from a GalNAc donor substrate (e.g. UDP-GalNAc) to a GalNAc acceptor containing a structure represented by formula 1 shown below to synthesize a compound containing a structure represented by formula 2 shown below, as well as the activity for transferring the GlcUA residue from a GlcUA donor substrate (e.g. UDP-GlcUA) to a GlcUA acceptor containing a structure represented by formula 3 shown below to synthesize a compound containing a structure represented by formula 4 shown below (see Reference Example 2 in this specification).

GlcUAβ1-(3GalNAcβ1-4GlcUAβ1)ₖ-(3GalNAc)₁ (1) (1)

GalNAcβ1-4GlcUAβ1-(3GalNAcβ1-4GlcUAβ1)ₖ-(3GalNAc)₁ (2) (2)

GalNAcβ1-(4GlcUAβ1-3GalNAcβ1)ₘ-(4GlcUA)ₙ (3) (3)

GlcUAβ1-3GalNAcβ1-(4GlcUAβ1-3GalNAcβ1)ₘ-(4GlcUA)ₙ (4) (4)

In formulas 1-4, "-" represents a glycoside linkage and the numerals designate the carbon positions in the saccharide ring at which said glycoside linkage is located; "α" and "β" refer to anomers having said glycoside linkage at 1-position of the saccharide ring, with "α" having a trans-configuration to CH₂OH or COOH at 5-position and "β" having a *cis*-configuration; k and m are each an integer of 1 or more; 1 and n are each 1 or 0.

The gene for the enzyme under consideration which comprised the nucleotide sequence of SEQ ID NO:1 was subjected to a locus search using OMIM (Online Mendelian Inheritance in Man) and it was found to exist at 5q31.1 (see Reference Example 1).

Another example of "glycosyltransferase 1" is a chondroitin synthase containing a protein comprising the amino acid sequence of amino acid Nos. 1-755 depicted in SEQ ID NO:4".

Said enzyme has the activity for transferring the GalNAc residue from a GalNAc donor (e.g. UDP-GalNAc) to a GalNAc acceptor containing a structure represented by formula 1 shown above to synthesize a compound containing a structure represented by formula 2 shown above, as well as the activity for transferring the GlcUA residue from a GlcUA donor (e.g. UDP-GlcUA) to a GlcUA acceptor containing a structure represented by formula 3 shown above to synthesize a compound containing a structure represented by formula 4 shown above (see Reference Example 4 in this specification).

The gene for the enzyme under consideration which comprised the nucleotide sequence of SEQ ID NO:3 was subjected to a locus search using OMIM and it was found to exist at 2q36.3 (see Reference Example 3 in this specification).

Speaking of the "activity for transferring a GlcUA residue or a GalNAc residue to the saccharide residue at the non-reducing terminal of chondroitin/chondroitin sulfate", a GlcUA donor substrate or a GalNAc donor substrate that are labeled, for example, with radioactivity and a non-radiolabeled acceptor substrate for chondroitin/chondroitin sulfate, which may be reduced in molecular weight to make lower molecular weight chondroitin/lower molecular weight chondroitin sulfate (sugar chains not longer than eicosasaccharide), may be used to perform enzymatic reaction and the activity under consideration can be easily detected by subjecting the reaction product to gel filtration and analysis with an autoradiograph or scintillation counter. The above-mentioned GlcUA donor substrate is preferably a sugar nucleotide having GlcUA and may be exemplified by adenosine diphosphate-D-glucuronic acid (ADP-GlcUA), uridine diphosphate-D-glucuronic acid (UDP-GlcUA), guanosine diphosphate-D-glucuronic acid (GDP-GlcUA), cytidine diphosphate-D-glucuronic acid (CDP-GlcUA), etc., with UDP-GlcUA being most preferred. For UDP-GlcUA works in vivo as a GlcUA donor substrate.

The above-mentioned GalNAc donor substrate is preferably a sugar nucleotide having GalNAc and may be exemplified by adenosine diphosphate-N-acetyl-D-galactosamine (ADP-GalNAc), uridine diphosphate-N-acetyl-D-galactosamine (UDP-GalNAc), guanosine diphosphate-N-acetyl-D-galactosamine (GDP-GalNAc), cytidine diphosphate-N-acetyl-D-galactosamine (CDP-GalNAc), etc., with UDP-GalNAc being most preferred. For UDP-GalNAc works *in vivo* as a GalNAc donor substrate.

The "glycosytransferase 2" mentioned above is not limited in any particular way as long as it is a glycosyltransferase which functions such that a xylose residue linked to an amino acid residue has D-galactose (hereunder also referred to simply as galactose or Gal) transferred thereto by a β1,4 glycoside linkage. Such glycosyltransferase 2 plays a role at the early stage of synthesis of chondroitin/chondroitin sulfate and if it mutates, normal synthesis of chondroitin/chondroitin sulfate is believed to be prevented, leading to Paget disease of bone which can cause abnormalities in bone formation.

The "amino acid residue" as it appears in the above definition of "glycosyltransferase 2" is preferably an amino acid that forms a peptide (protein), most preferably an amino acid having linked thereto xylose which is present at the reducing terminal of a characteristic tetrasaccharide structure (GlcUAβ1-3Galβ1-3Galβ1-4Xyl: this is hereunder designated "linkage tetrasaccharide") that exists at the linkage between a peptide (protein) and chondroitin/chondroitin sulfate in the structure of an in vivo chondroitin/chondroitin sulfate proteoglycan. The most preferred example of such amino acid is L-serine.

In short, "glycosyltransferase 2" has the activity for transferring a Gal residue from a Gal donor substrate (e.g. UDP-Gal) to a Gal acceptor containing a structure represented by formula 5 shown below to synthesize a compound containing a structure represented by formula 6 shown below (see Reference Example 5 in this specification).

Xylβ1-Ser (5) (5)

Galβ1-4Xylβ1-Ser (6) (6)

In above formulas 5 and 6, "Ser" represents a serine residue (which may be located in a peptide chain) having linked to its side chain a xylose residue represented by the above "Xyl"; "Xyl" represents a xylose residue linked to the side chain of a serine residue represented by said "Ser"; '-", "β" and the numerals have the same meanings as defined for the above formulas 1-4.

The gene for the enzyme under consideration which comprised the nucleotide sequence (SEQ ID NO:5) was subjected to a locus search using OMIM and it was found to exist at 5q35 (see Reference Example 5 in the present specification).

Speaking of the activity such that the xylose residue linked to an amino acid residue has Gal transferred thereto by a β1,4 glycoside linkage, a Gal donor substrate that is labeled, for example, with radioactivity and a non-radiolabeled xylose linked peptide (e.g. the N-terminal sequence of bikunin having xylose linked as a side chain to a serine residue which is the 9th amino acid residue in SEQ ID NO:15; produced by Peptide Institute) may be used to perform reaction and the activity under consideration can be easily detected by subjecting the reaction product to gel filtration and analysis with an autoradiograph or scintillation counter. The above-mentioned Gal donor substrate is preferably a sugar nucleotide having Gal and may be exemplified by adenosine diphosphate-D-galactose (ADP-Gal), uridine diphosphate-D-galactose (UDP-Gal), guanosine diphosphate-D-galactose (GDP-Gal), cytidine diphosphate-D-galactose (CDP-Gal), etc., with UDP-Gal being most preferred. For UDP-Gal works *in vivo* as a Gal donor substrate.

Speaking of gene mutations in Paget disease of bone, it is known that in subspecies PDB2, PDB3 and PDB4, mutation has occurred at 18q22.1, 5q35 and 5q31, respectively (OMIM #602080). It is also known that gene mutations in familial Paget disease of bone are at 2q36, 10q13 and 5q35 (Am. J. Hum. Genet. 69(2001), pp. 1055-1061). In these known sites of mutation, several to ten-odd genes are coded and the positions at which the aforementioned chondroitin synthase genes are located are 5q31.1 for SEQ ID NO:1 (k3), 2q36.3 for SEQ ID NO:3 (k11) and 5q35 for SEQ ID NO:5 (β4GalT7), which are respectively in complete agreement with the positions where the above-mentioned mutations occur. Thus, all genes for those enzymes described above that participate in the synthesis of chondroitin/chondroitin sulfate overlap the sites of gene mutations in Paget disease of bone, so the probability is extremely high that the mutations of those genes will cause disorder in the biosynthesis of chondroitin/chondroitin sulfate, eventually leading to the manifestation of Paget disease of bone.

The expression which reads "associating Paget disease of bone with the mutation of a gene coding a chondroitin synthase or the amount of expression of said gene", as used in the present specification means that a detection of a mutation in a chondroitin synthase gene or an amount of expression of that gene which is lower than the value for a normal subject is diagnosed as Paget disease of bone.

The term "gene mutation" as used in the present specification means a structural change in gene and "mutation" encompasses point mutation, inversion, deletion, insertion, duplication and translocation. A "mutant gene" means a nucleotide sequence as depicted in SEQ ID NO: 1, SEQ ID NO: 3, SEQ ID NO: 5, SEQ ID NO: 65, SEQ ID NO: 67 or SEQ ID NO: 69, provided that one or more nucleotides are replaced or deleted or that one or more nucleotides are inserted or added to said nucleotide sequence. The term "more" as used herein preferably means at least 2, more preferably at least 4, even more preferably at least 6, and most preferably at least 10.

Modes of mutation in a chondroitin synthase gene are preferably point mutation, deletion, insertion, inversion, replication and translocation, more preferably point mutation, deletion, insertion, inversion and translocation, even more preferably point mutation, deletion, insertion and translocation, and most preferably point mutation, deletion and insertion.

The term "gene expression" as used in the present specification typically means the transcription from DNA or a portion thereof to RNA or the translation from said RNA or a portion thereof to a protein. Therefore, when we say "the amount of gene expression", we mean the amount of RNA transcribed from a gene or the amount of a protein translated from said RNA.

The amount of RNA expression can be determined, for example, by PCR analysis using the primers mentioned in Example 1 to be described later. For PCR analysis, the use of a quantitative PCR assay is preferred, and RT-PCR assay and quantitative real-time PCR assay may be given as examples for kinetics analysis. According to the present invention, these are not the only methods for quantitating RNA and Northern blot, dot blot and DNA microarrays can also be employed. If desired, nucleic acids of genes that commonly occur widely in the same tissue and the like, for example, nucleic acids that code for glyceraldehyde 3-phosphate dehydrogenase (GAPDH) and β-actin can be used as control. In addition, the amount of protein expression can be determined by, for example, ELISA and Western blot.

The detection method of the present invention can specifically be performed in the following way.

Namely, from a gene-containing specimen (which may be an epithelial tissue, a connective tissue, etc., preferably, a connective tissue, and a particularly preferred example is blood since it is extremely easy to sample and the specimen obtained is fresh enough), total RNA can be extracted and purified using, for example, an existing RNA preparing kit (e.g. RNA Extraction Kit of Amersham Biosciences K.K.) Using the extracted and purified total RNA, the amounts of K3, K11 and β4Gal-T7 expressed can be determined by RT-PCR and other methods using an existing kit (e.g. SUPERSCRIPT First-Strand Synthesis System for RT-PCR of Invitrogen Co.) Exemplary combinations of 5'-primer, 3'-primer and probe that can be employed include the combination of SEQ ID NO:56, SEQ ID NO:57 and SEQ ID NO:58 for the case of determining the amount of K3 expression, the combination of SEQ ID NO:59, SEQ ID NO:60 and SEQ ID NO:61 for the case of determining the amount of K11 expression, and the combination of SEQ ID NO:62, SEQ ID NO:63 and SEQ ID NO:64 for the case of determining the amount of βGal-T7 expression. It should be noted here that a skilled artisan can choose and prepare suitable combinations as appropriate for the specific purpose of quantification.

Then, a patient who, as the result of those quantifications, has been found to have the expression of any one of the genes K3, K11 and β4GalT7 suppressed either partly or completely can be diagnosed as a patient having high risk for Paget disease of bone. As also noted above, those quantifications can be effected by determining the amount of the expressed gene K3, K11 or β4Gal-T7 from the quantity of RNA; alternatively, an antibody against K3, K11 or β4Gal-T7 may be prepared in accordance with a conventional technique and it may be used to determine directly the quantity of the protein from K3, K11 or β4Gal-T7; yet alternatively, the activity of any one of those enzymes in a specimen may be directly measured to determine the quantity of that enzyme so that it is associated with Paget disease of bone.

More specifically, as will be mentioned in Example 2 to be described later, the ratio in the amount of expression of a chondroitin synthase gene in peripheral blood-derived mononuclear cells from a patient with Paget disease of bone and in normal peripheral blood-derived mononuclear cells may be measured by quantitative PCR to evaluate for Paget disease of bone. According to the present invention, the ratio in the amount of gene expression that can be relied upon to evaluate for Paget disease of bone is 1/2 or less, preferably 1/5 or less, and more preferably 1/10 or less. It should also be noted that the term "measurement" as used in the present invention covers any one of detection, amplification, quantification and semi-quantification.

According to the present invention, Paget disease of bone can be detected by confirming the presence of a mutation in a chondroitin synthase gene. To be specific, the nucleotide sequence of a chondroitin synthase gene extracted from mononuclear cells in the peripheral blood of a patient with Paget disease of bone is compared with the nucleotide sequence of the chondroitin synthase gene extracted from mononuclear cells in normal peripheral blood and the detection of any mutation can be identified as indicative of Paget disease of bone. To be more specific, as will be set forth in Example 1 to be described later, primers prepared appropriately on the basis of the nucleotide sequences of K3, K11 and β4Gal-T7 described in the Sequence Listing as accompanying the present specification are used to amplify the gene encoding K3, K11 or β4Gal-T7 directly from genomic DNA by a suitable technique such as PCR assay and its nucleotide sequence is analyzed in accordance with a known nucleotide sequencing method, with the presence of any mutation in one of those genes being held as indicative of Paget disease of bone. The number of mutant nucleotides that can be considered to be indicative of Paget disease of bone is at least 1, preferably at least 4, more preferably at least 6, and most preferably at least 10.

Modes of mutation in the chondroitin synthase genes are preferably point mutation, deletion, insertion, inversion, replication and translocation, more preferably point mutation, deletion, insertion, inversion and translocation, even more preferably point mutation, deletion, insertion and translocation, and most preferably point mutation, deletion and insertion.

Now that it has become clear according to the present invention that K3, K11 and β4Gal-T7 are associated with Paget disease of bone, a knockout animal which shows the pathology of Paget disease of bone can be prepared by suppressing the expression of those genes either partly or completely in a laboratory animal. Preferred knockout animals are mammals including mouse, rat, rabbit, dog, cat and monkey, with mouse and rat being most preferred since the techniques of preparing knockout animals from them have been established to some extent; however, those are not the sole examples of the knockout animals that can be prepared in the present invention.

For instance, knockout mice (pathological model mice) that show the pathology of Paget disease of bone can be prepared in accordance with the descriptions in "Latest Technology of Gene Targeting" (ed. by T. Yagi, Yodosha Co., LTD.) and "Gene Targeting" (translated and supervised by T. Noda, Medical Sciences International, LTD.)

Suppose, for instance, that a mouse gene to be knocked out is K3 (mK3: SEQ ID NO:65), K11 (mK11: SEQ ID NO:67) or β4Gal-T7 (mβ4Gal-T7: SEQ ID NO:69). A linear targeting vector (80 µg) with an insert of chromosomal fragment (ca. 10 kb) centering on an exon that contains a domain for activating that gene (a fragment containing the third exon (1560 bp) in the ORF portion of mK3; a fragment containing 4598 bp corresponding to the full length of ORF in mK11; and a fragment containing the second to sixth exons of ORF in β4Gal-T7) is introduced into ES cells (derived from E14/129Sv mouse) by a known method such as electroporation; G418 resistant colonies are selected, transferred to a 24-well plate and cultivated; after storing some of the cells in a frozen state, DNA is extracted from the remaining ES cells and about 120 colonies of clones that are found to have undergone recombination are selected by a known nucleotide sequence analyzing method; further, Southern blotting and other suitable methods are employed to confirm that recombination has taken place as intended; finally, about 10 recombinant clones are selected and ES cells from about two of them are injected into blastocytes of C57BL/6 mouse; the mouse embryo into which the ES cells were thusly injected is transplanted into the womb of a surrogate mouse which will then give birth to a chimera mouse; a hetero knockout mouse can be obtained by subjecting the chimera mouse to germ transmission.

A knockout mouse can also be prepared by a method for suppressing gene expression, such as the small interfering RNA assay (T.R. Brummelkamp et al., Science, 296, 550-553 (2002)).

More specifically, as set forth in Example 3 to be described later, a knockout mouse can be prepared by first transferring a chondroitin synthase gene into ES cells and then using clones that have undergone the intended homologous recombination to establish a mouse lineage in a reproductive cell line.

### Examples

On the pages that follow, the present invention is described more specifically by means of Examples.

### Reference Example 1: Preparation of Chondroitin Sulfate Synthase (K3)

### (1) Cloning of cDNA and constructing an expression vector

BLAST search was performed using as a query the amino acid sequence of β1,4-galactose transferase (β4Gal-T) (the amino acid sequence encoded by GenBank accession No. D29805). As the result, Expression Sequence Tag (EST: GenBank accession No. AC0004219) was discovered. Since this sequence was incomplete, ORF was investigated from a genomic database by means of GenScan (Stanford University, USA). As the result, the nucleotide sequence depicted in SEQ ID NO: 1 (encoding the amino acid sequence of SEQ ID NO: 2) was discovered. The gene comprising the nucleotide sequence depicted in SEQ ID NO: 1 is at least expressed in the human brain and this was verified by RT-PCR assay using Marathon-Ready cDNA (CLONTECH Laboratories, Inc.) as a template (as PCR primers, the combinations of 5' primer and 3' primer according to SEQ ID NO: 9 and SEQ ID NO: 10, as well as SEQ ID NO: 11 and SEQ ID NO: 12 were used). In order to clone a soluble region of the gene product of interest other than a region including the transmembrane region (the region to be excluded comprising amino acid Nos. 1-129 in SEQ ID NO: 2), PCR was performed according to a conventional technique using the two primers of SEQ ID NO: 7 and SEQ ID NO: 8. The template cDNA used was Marathon-Ready cDNA human brain (CLONTECH Laboratories, Inc.) The amplified band of about 2.3 kb was digested with HindIII and XbaI according to a conventional technique and inserted between HindIII and XbaI sites of a mammalian cell expression vector pFLAG-CMV-1 (Sigma) according to a conventional technique, thereby making an expression vector (K3-FLAG-CMV1). Nucleotide sequencing of the obtained expression vector showed the insertion of a DNA fragment comprising a nucleotide sequence of nucleotide Nos. 388-2649 in the nucleotide sequence depicted in SEQ ID NO: 1. The genomic position of DNA having that nucleotide sequence was identified by using OMIM and it was found to exist at 5q31.1.

### (2) Preparing K3

K3-FLAG-CMV1 (15 µg) was set on TransFast (Promega Corp.) which was operated according to the protocol to transfer the gene into COS7 cells that had been cultivated on 100 mm culture dishes to produce 70% confluent cultures. After 3-day cultivation, the supernatant was recovered and passed through a 0.22 µm filter; thereafter, 100 µl of Anti-FLAG M2-Agarose Affinity Gel (Sigma) was added to 10 ml of the supernatant and mixing by inverting of the tube was performed overnight at 4°C. After the reaction, the gel was washed three times with 50 mM Tris-HCl (pH 7.4)/20% glycerol and the unwanted wash solution was removed with a syringe fitted with a 27G needle. The gel was suspended in50 mmol/L Tris-HCl at pH 7.4 (20 glycerol, 10 mmol/L phenylmethyl sulfonyl fluoride, 1 µg/ml leupeptin and 1 µg/ml pepstatin) to make 50 (v/v); after centrifugation, the supernatant was removed to prepare an enzyme adsorbed gel suspension.

### Reference Example 2

### Extension of Chondroitin Building Blocks Using K3

### (1) Preparation of chondroitin/chondroitin sulfate odd-numbered saccharides

Chondroitin (shark cartilage derived chondroitin sulfate as chemically desulfurylated: product of Seikagaku Corporation) and chondroitin sulfate (as derived from shark cartilage: product of Seikagaku Corporation: also called chondroitin sulfate C) were subjected to limited digestion with bovine testis hyaluronidase (Sigma) and thereafter the reaction solution was held at 100°C for 10 minutes until the enzyme was deactivated thermally. The reaction solution was loaded on a Superdex 30 column (60 x 1.6 cm: product of Amersham Biosciences K.K.; chromatographic conditions: mobile phase, 0.2 mol/L NH₄HCO₃; flow rate, 2 ml/min) and the effluent was fractionated for each 2 ml with monitoring at an absorbance of 225 nm, followed by pooling of fractions equivalent to decasaccharide. Those fractions were desalted by PD10 column (Amersham Biosciences K.K.) and after uronic acid was quantitated by the carbazole sulfate method according to a conventional technique, the fractions were freeze-dried. The freeze-dried product was dissolved in distilled water to give 1 mM, thereby preparing even-numbered oligosaccharide samples (chondroitin derived decasaccharide is hereunder designated CH10, and chondroitin sulfate derived decasaccharide as CS10).

To 50 mmol/L MES buffer solution (pH 6.5) containing 10 nmol/L MnCl₂ and 171 µmol/L ATP sodium salt, 10 µl of the enzyme adsorbed gel suspension, 1 nmol of a test substance (chondroitin (CHEL), chondroitin sulfate (CSEL), CH10 or CS10) and 0.036 nmol of [³H]UDP-GalNAc were added to make a total volume of 30 µl. Enzymatic reaction was carried out at 37°C for 1 hour and thereafter the reaction solution was kept at 100°C for 1 minute until the enzyme was deactivated for quenching the reaction.

Each of the reaction solutions was passed through a microfilter with a pore size of 0.22 µm (Millipore Corp.); thereafter, it was separated by a Superdex peptide column (30 x 1.0 cm: product of Amersham Biosciences K.K.; chromatographic conditions: mobile phase, 0.2 mol/L NaCl; flow rate, 1.0 ml/min) and the effluent was recovered at 0.5 ml fractions, followed by radioactivity measurement with a scintillation counter (Fig. 1). As the result, strong GalNAc transfer activity was observed when CHEL (fractions 17-18), CH10 (fraction 23) and CS10 (fraction 23) were used as a GalNAc acceptor substrate but no GalNAc transfer activity was observed with CSEL (fraction 16). Fraction 23 as each of the reaction products from CH10 and CS10 showed a molecular weight at which undecasaccharide was eluted. The undecasaccharide obtained from CH10 was designated CH11(K3) and the one obtained from CS10 designated as CS11(K3).

Fractions 21-25 of CS11(K3) were recovered, pooled and desalted with a PD10 column. The thus obtained sample was divided into two equal portions and freeze-dried. One of the two halved portions was dissolved in 100 µl of 0.1 mol/L Tris-HCl buffer solution (pH 7.4) containing 30 mM sodium acetate; the resulting solution was designated CS11(K3)A. The other portion was digested with chondroitinase ACII (100 mU of chondroitinase ACII (Seikagaku Corporation) was dissolved in 100 µl of CS11(K3) fraction, digested enzymatically at 37°C for 10 hours, and heated to deactivate the enzyme; the resulting product was designated CS11(K3)B).

CS11(K3)A and CS11(K3)B were passed through a microfilter with a pore size of 0.22 µm (Millipore Corp.); thereafter, they were separated by a Superdex peptide column (30 x 10 mm: product Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter to give a radioactivity peak shift to a monosaccharaide fraction in CS11(K3)B (Fig. 2). From this result, it became clear that K3 transferred GalNAc to GlcUA at the non-reducing terminal of the chondroitin sulfate derived decasaccharide via β1,4 linkage to prepare an undecasaccharide.

### (2) Preparation of chondroitin/chondroitin sulfate even-numbered saccharides

Chondroitin (shark cartilage derived chondroitin sulfate as chemically desulfurylated: product of Seikagaku Corporation) and chondroitin sulfate (as derived from shark cartilage: product of Seikagaku Corporation: also called chondroitin sulfate C) were subjected to limited digestion with bovine testis hyaluronidase (Sigma) and thereafter the reaction solution was held at 100°C for 10 minutes until the enzyme was deactivated thermally. The reaction solution was centrifuged at 10,000xg for 10 minutes and the recovered supernatant was further digested with bovine liver derived β glucuronidase (Sigma). The enzymatic reaction was quenched by keeping the reaction solution at 100°C for 10 minutes. The reaction solution was then loaded on a Superdex 30 column (60 x 1.6 cm: product of Amersham Biosciences K.K.; chromatographic conditions: mobile phase, 0.2 mol/L NH₄HCO₃; flow rate, 2 ml/min) and the effluent was fractionated for each 2 ml with monitoring at an absorbance of 225 nm, followed by pooling of fractions equivalent to undecasaccharide. Those fractions were desalted by PD10 column (Amersham Biosciences K.K.) and after uronic acid was quantitated by the carbazole sulfate method according to a conventional technique, the fractions were freeze-dried. The freeze-dried product was dissolved in distilled water to give 1 mmol/L, thereby preparing odd-numbered oligosaccharide samples (chondroitin derived undecasaccharide is hereunder designated CH11, and chondroitin sulfate derived decasaccharide as CS11).

In addition, chondroitin (shark cartilage derived chondroitin sulfate as chemically desulfurylated: product of Seikagaku Corporation) and chondroitin sulfate (as derived from shark cartilage: product of Seikagaku Corporation: also called chondroitin sulfate C) were digested with bovine liver derived β glucuronidase (Sigma) to prepare samples (which are respectively degignated CHOL and CSOL).

To 50 mmol/L acetate buffer solution (pH 5.6) containing 10 mmol/L MnCl₂, 10 µl of the enzyme adsorbed gel suspension, 1 nmol of a test substance (CHOL, CSOL, CH11 or CS11) and 0.432 nmol of [¹⁴C]UDP-GlcUA were added to make a total volume of 30 µl. Enzymatic reaction was carried out at 37°C for 1 hour and thereafter the reaction solution was kept at 100°C for 1 minute until the enzyme was deactivated for quenching the reaction.

Each of the reaction solutions was passed through a microfilter with a pore size of 0.22 µm (Millipore Corp.); thereafter, it was separated by a Superdex peptide column (30 x 1.0 cm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter (Fig. 3). As the result, strong GlcUA transfer activity was observed when CHOL (fractions 17-18), CH11 (fraction 23) and CS11 (fraction 23) were used as a GlcUA acceptor substrate but no GlcUA transfer activity was observed with CSOL (fraction 16). Fractions 22-23 as the reaction products from CH11 and CS11 showed molecular weights at which dodecasaccharide was eluted. The dodecasaccharide obtained from CH11 was designated CH12(K3) and the one obtained from CS11 designated as CS12(K3).

Fractions 21-25 of CS12(K3) were recovered, pooled and desalted with a PD10 column. The thus obtained sample was divided into two equal portions and freeze-dried. One of the two halved portions was dissolved in 100 µl of 0.1 mol/L Tris-HCl buffer solution (pH 7.4) containing 30 mmol/L sodium acetate; the resulting solution was designated CS12(K3)A. The other portion was digested with chondroitinase ACII (100 munits of chondroitinase ACII (Seikagaku Corporation) was dissolved in 100 µl of CS11(K3) fraction, digested enzymatically at 37°C for 10 hours, and heated to deactivate the enzyme; the resulting product was designated CS12(K3)B).

CS12(K3)A and CS12(K3)B were passed through a microfilter with a pore size of 0.22 µm (Millipore Corp.); thereafter, they were separated by a Superdex peptide column (30 x 1.0 cm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter to give a radioactivity peak shift to a disaccharaide fraction in CS12(K3)B (Fig. 4). From this result, it became clear that K3 transferred GlcUA to the chondroitin sulfate derived undecasaccharide via β1,3 linkage to prepare a dodecasaccharide.

### Reference Example 3: Preparation of Chondroitin Sulfate Synthase (K11)

### (1) Cloning of cDNA and constructing an expression vector

BLAST search was performed using as a query the amino acid sequence of chondroitin sulfate glucuronic acid transferase (CSGlcA-T) (the amino acid sequence encoded by GenBank accession No. AB037823). As the result, EST (GenBank accession NM_018590) was discovered. Since this sequence was incomplete, ORF was investigated from a genomic database by means of GenScan (Stanford University, USA). As the result, the nucleotide sequence depicted in SEQ ID NO: 3 (encoding the amino acid sequence of SEQ ID NO: 4) was discovered. The gene comprising the nucleotide sequence depicted in SEQ ID NO: 3 is at least expressed in the human brain and this was verified by RT-PCR assay using Marathon-Ready cDNA (CLONTECH Laboratories, Inc.) as a template. In order to clone a soluble region of the gene of interest other than a region including the transmembrane region (the region to be excluded comprising amino acid Nos. 1-96 in SEQ ID NO: 4), PCR was performed according to a conventional technique using the two primers of SEQ ID NO: 13 and SEQ ID NO: 14. The template cDNA used was Marathon-Ready cDNA human brain (CLONTECH Laboratories, Inc.) The amplified band of about 2 kb was digested with EcoRI and BamHI according to a conventional technique and inserted between EcoRI and BamHI sites of a mammalian cell expression vector pFLAG-CMV-1 (Sigma) according to a conventional technique, thereby making K11-FLAG-CMV1. Nucleotide sequencing of the obtained vector showed the insertion of a DNA fragment comprising a nucleotide sequence of nucleotide Nos. 287-2328 in the nucleotide sequence depicted in SEQ ID NO: 3. The genomic position of DNA having that nucleotide sequence was identified by using OMIM and it was found to exist at 2q36.3.

### (2) Preparing K11

K11-FLAG-CMV1 (15 mg) was set on TransFast (Promega Corp.) which was operated according to the protocol to transfer the gene into COS7 cells that had been cultivated on 100 mm culture dishes to produce 70% confluent cultures. After 3-day cultivation, the supernatant was recovered and passed through a 0.22 µm filter; thereafter, 100 µl of Anti-FLAG M2-Agarose Affinity Gel (Sigma) was added to 10 ml of the supernatant and mixing by inverting of the tube was performed overnight at 4°C. After the reaction, the gel was washed three times with 50 mmol/L Tris-HCl at pH 7.4 (20 glycerol) and the unwanted wash solution was removed with a syringe fitted with a 27G needle. The gel was suspended in 50 mmol/L Tris-HCl at pH 7.4 (20% glycerol, 10 mmol/L phenylmethyl sulfonyl fluoride, 1 µg/ml leupeptin and 1 µg/ml pepstatin) to make 50 (v/v); after centrifugation, the supernatant was removed to prepare an enzyme adsorbed gel suspension.

### Reference Example 4

### Extension of Chondroitin Building Blocks Using K11

### (1) Preparation of chondroitin/chondroitin sulfate odd-numbered saccharides

To 50 mmol/L MES buffer solution (pH 6.5) containing 10 nmol/L MnCl₂ and 171 µmol/L ATP sodium salt, 10 µl of the enzyme adsorbed gel suspension, 1 nmol of a test substance (CHEL, CSEL, CH10 or CS10) and 0.036 nmol of [³H]UDP-GalNAc were added to make a total volume of 30 µl. Enzymatic reaction was carried out at 37°C for 1 hour and thereafter the reaction solution was kept at 100°C for 1 minute until the enzyme was deactivated for quenching the reaction.

Each of the reaction solutions was passed through a microfilter with a pore size of 0.22 µm (Millipore Corp.); thereafter, it was separated by a Superdex peptide column (30 x 1.0 cm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter (Fig. 5). As the result, strong GalNAc transfer activity was observed when CHEL (fraction 18), CH10 (fraction 23) and CS10 (fraction 23) were used as a GalNAc acceptor substrate and weak GalNAc transfer activity was observed with CSEL (fraction 16). Fractions 22-23 as the reaction products from CH10 and CS10 showed molecular weights at which undecasaccharide was eluted. The undecasaccharide obtained from CH10 was designated CH11(K11) and the one obtained from CS10 designated as CS11(K11).

Fractions 21-25 of CS11(K11) were recovered, pooled and desalted with a PD10 column. The thus obtained sample was divided into two equal portions and freeze-dried. One of the two halved portions was dissolved in 100 µl of 0.1 mol/L Tris-HCl buffer solution (pH 7.4) containing 30 mM sodium acetate; the resulting solution was designated CS11(K11)A. The other portion was digested with chondroitinase ACII (100 munits of chondroitinase ACII (Seikagaku Corporation) was dissolved in 100 µl of CS11(K11) fraction, digested with an enzyme at 37°C for 10 hours, and heated to deactivate the enzyme; the resulting product was designated CS11(K11)B).

CS11(K11)A and CS11(K11)B were passed through a microfilter with a pore size of 0.22 µm (Millipore Corp.); thereafter, they were separated by a Superdex peptide column (30 x 10 mm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter to give a radioactivity peak shift to a trisaccharaide fraction in CS11(K11)B (Fig. 6). From this result, it became clear that K11 transferred GalNAc to GlcUA at the non-reducing terminal of the chondroitin sulfate derived decasaccharide via β1,4 linkage to prepare an undecasaccharide.

### (2) Preparation of chondroitin/chondroitin sulfate even-numbered saccharides

To 50 mmol/L acetate buffer solution (pH 5.6) containing 10 nmmol/L MnCl₂, 10 µl of the enzyme adsorbed gel suspension, 1 nmol of a test substance (CHOL, CSOL, CH11 or CS11) and 0.432 nmol of [¹⁴C]UDP-GlcUA were added to make a total volume of 30 µl. Enzymatic reaction was carried out at 37°C for 1 hour and thereafter the reaction solution was kept at 100°C for 1 minute until the enzyme was deactivated for quenching the reaction.

Each of the reaction solutions was passed through a microfilter with a pore size of 0.22 µm (Millipore Corp.); thereafter, it was separated by a Superdex peptide column (30 x 1.0 cm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter (Fig. 3). As the result, strong GlcUA transfer activity was observed when CHOL (fractions 18), CH11 (fraction 23) and CS11 (fraction 22) were used as a GlcUA acceptor substrate but no GlcUA transfer activity was observed with CSOL. Fractions 22-23 as the reaction products from CH11 and CS11 showed molecular weights at which dodecasaccharide was eluted. The dodecasaccharide obtained from CH11 was designated CH12(K11) and the one obtained from CS11 designated as CS12(K11).

Fractions 21-25 of CS12(K11) were recovered, pooled and desalted with a PD10 column. The thus obtained sample was divided into two equal portions and freeze-dried. One of the two halved portions was dissolved in 100 µl of 0.1 mol/L Tris-HCl buffer solution (pH 7.4) containing 30 mmol/L sodium acetate; the resulting solution was designated CS12(K11)A. The other portion was digested with chondroitinase ACII (100 munits of chondroitinase ACII (Seikagaku Corporation) was dissolved in 100 µl of CS11(K11) fraction, digested enzymatically at 37°C for 10 hours, and heated to deactivate the enzyme; the resulting product was designated CS12(K11)B).

CS12(K11)A and CS12(K11)B were passed through a microfilter with a pore size of 0.22 µm (Millipore Corp.); thereafter, they were separated by a Superdex peptide column (30 x 1.0 cm: product of Amersham Biosciences K.K.; chromatographic conditions: mobile phase, 0.2 mol/L NaCl; flow rate, 0.5 ml/min) and the effluent was recovered at 0.5 ml fractions, followed by radioactivity measurement with a scintillation counter to give a radioactivity peak shift to a disaccharaide fraction in CS12(K11)B (Fig. 4). From this result, it became clear that the enzyme of the present invention could transfer GlcUA to the chondroitin sulfate derived undecasaccharide via β1,3 linkage to prepare a dodecasaccharide.

### Reference Example 5

### Preparation of Chondroitin Sulfate Synthase (β4Gal-T7) and Verification of Its Activity

Cloning of β4Gal-T7 was performed according to the method of Almeida et al. (EXPERIMENTAL PROCEDURES in J. Biol. Chem., 274, 37, 26165-26171 (1999)). The nucleotide sequence of the clones obtained was verified by a conventional technique and it was found to comprise the nucleotide sequence depicted in SEQ ID NO: 5. The genomic position of DNA having that nucleotide sequence was identified by using OMIM and it was found to exist at 5q35.

Further, in accordance with the method of Gotoh et al. (J. Biol. Chem., 277, 41, 38189-38196 (2002)), β4Gal-T7 was expressed and there was prepared a suspension of an affinity carrier having the β4Gal-T7 linked thereto; thereafter, 10 µl of the gel, 6.25 mg of a acceptor substrate (xylose linked peptide [the N-terminal sequence of bikunin having xylose linked as a side chain to serine which was the amino acid residue at 9-position of SEQ ID NO: 15]: product of Peptide Institute), 171 µmol of adenosine triphosphate (ATP) sodium salt and 0.036 mol of [³H]UDP-Gal were added to 50 mmol of MES buffer solution (pH 6.5) containing 10 nmol/L MnCl₂ to make a total volume of 30 µl and reaction was carried out at 37°C for 1 hour. Thereafter, the reaction solution was kept at 100°C for 1 minute until the enzyme was deactivated for quenching the reaction.

The reaction solution was passed through a microfilter with a pore size of 0.22 µm (Millipore Corp.); thereafter, it was separated by a Superdex peptide column (30 x 1.0 cm: product of Amersham Biosciences K.K.; chromatographic conditions: mobile phase, 0.2 mol/L NaCl; flow rate, 1.0 ml/min) and the effluent was recovered at 0.5 ml fractions, followed by radioactivity measurement with a scintillation counter. As the result, strong radioactivity was observed in a polymer fraction other than the eluted fraction of [³H]UDP-Gal. This supported the linking by β4Gal-T7 of galactose to the acceptor substrate. Example 1 Extraction of DNA From Blood Sample and Analysis of Its Nucleotide Sequence

DNA was extracted from 1 ml of blood using GFX Genomic Blood DNA Purification Kit (Amersham Biosciences K.K.) Of the 14-16 µg of DNA obtained, 250 ng was used as a template to amplify the exons in the genes of chondroitin sulfate synthases (K3, K11, β4Gal-T7) with the aid of various primers (see Table 1 below). Using the thus obtained fragments as templates, the nucleotide sequences of the genes were analyzed by a conventional technique and compared with those of the genes of normal types of the respective chondroitin synthases; in this way, one could easily check for the presence of mutations and single nucleotide polymorphisms.

**Table 1**

| | For exon amplification | | For nucleotide sequence analysis |
|---|---|---|---|
| | 5'primer | 3'primer | |
| K3 (Exon 1) | SEQ ID NO:16 | SEQ ID NO:17 | SEQ ID NO:16, SEQ ID NO:17, SEQ ID NO:18, SEQ ID NO:19 |
| K3 (Exon 2) | SEQ ID NO:20 | SEQ ID NO:21 | SEQ ID NO:20, SEQ ID NO:21 |
| K3 (Exon 3) | SEQ ID NO:22 | SEQ ID NO:23 | SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31 |
| K11 (Exon 1) | SEQ ID NO:32 | SEQ ID NO:33 | SEQ ID NO:32, SEQ ID NO:33 |
| K11 (Exon 2) | SEQ ID NO:34 | SEQ ID NO:35 | SEQ ID NO:34, SEQ ID NO:35 |
| K11 (Exon 3) | SEQ ID NO:36 | SEQ ID NO:37 | SEQ ID NO:36, SEQ ID NO:37 |
| K11 (Exon 4) | SEQ ID NO:38 | SEQ ID NO:39 | SEQ ID NO:38, SEQ ID NO:39, SEQ ID NO:40, SEQ ID NO:41, SEQ ID NO:42, SEQ ID NO:43 |
| β4Gal-T7 (Exon 1) | SEQ ID NO:44 | SEQ ID NO:45 | SEQ ID NO:44, SEQ ID NO:45 |
| β4Gal-T7 (Exon 2) | SEQ ID NO:46 | SEQ ID NO:47 | SEQ ID NO: 46, SEQ ID NO:47 |
| β4Gal-T7 (Exon 3) | SEQ ID NO:48 | SEQ ID NO:49 | SEQ ID NO:48, SEQ ID NO:49 |
| β4Gal-T7 (Exon 4) | SEQ ID NO:50 | SEQ ID NO:51 | SEQ ID NO:50, SEQ ID NO:1 |
| β4Gal-T7 (Exon 5) | SEQ ID NO:52 | SEQ ID NO:53 | SEQ ID NO:52, SEQ ID NO:53 |
| β4Gal-T7 (Exon 6) | SEQ ID NO:54 | SEQ ID NO:55 | SEQ ID NO:54, SEQ ID NO:55 |

### Example 2 Extraction of RNA From Blood Sample and Analysis of the Amount Expressed

A blood sample (10 ml) was centrifuged at 3,000 rpm for 10 minutes and the intermediate layer was separated off. Further centrifugation was performed using Lympho Prep (Nycomed Inc.) and the intermediate layer was separated off. By these procedures, 9 x 10⁷ peripheral blood-derived mononuclear cells (PBMC) were obtained. From all quantities of PBMC, whole RNA can be prepared using RNA Extraction Kit (Amersham Biosciences K.K.) From 10 ng of the whole RNA, 1st strand cDNA was synthesized by SUPERSCRIPT First-Stand Synthesis System for RT-PCR (Invitrogen Co.) Using this cDNA as a template, real-time PCR assay was performed to determine the amounts of various genes expressed. The primers and probes used are shown in Table 2.

**Table 2**

| | 5'Primer | 3'Primer | Probe |
|---|---|---|---|
| K3 | SEQ ID NO:56 | SEQ ID NO:57 | SEQ ID NO:58 |
| K11 | SEQ ID NO:59 | SEQ ID NO:60 | SEQ ID NO:61 |
| β4Gal-T7 | SEQ ID NO:62 | SEQ ID NO:63 | SEQ ID NO:64 |

### Example 3 Preparation of Pathogenic Model Mouse

Knockout mice were obtained by the following procedure using a mouse gene K3 (mK3: SEQ ID NO:65), K11 (mK11: SEQ ID NO:67) or β4Gal-T7 (mβ4Gal-T7: SEQ ID NO:69). A linear targeting vector (80 µg) with an insert of chromosomal fragment (ca. 10 kb) centering on an exon that contained a domain for activating that gene (a fragment containing the third exon (1560 bp) in the ORF portion in the case of mK3; a fragment containing 4598 bp corresponding to the full length of ORF in mK11; and a fragment containing the second to sixth exons of ORF in β4Gal-T7) was introduced into ES cells (derived from E14/129Sv mouse) by electroporation; G418 resistant colonies were selected; the G418 resistant colonies were transferred to a 24-well plate and cultivated; after storing some of the cells in a frozen state, DNA was extracted from the remaining ES cells and about 120 colonies of clones that were found to have undergone recombination were selected by PCR; further, Southern blotting was employed to confirm that recombination had taken place as intended; finally, about 10 recombinant clones were selected and ES cells from about two of them were injected into blastocytes of C57BL/6 mouse; the mouse embryo into which the ES cells were thusly injected was transplanted into the womb of a surrogate mouse which then gave birth to a chimera mouse; by subsequent germ transmission, a hetero knockout mouse was obtained.

### Reference Documents

1. J. Bone Miner Res., 11(1996), pp. 1602-1607
2. Endocr. Metab. Clin. North. Am., 24(1995), pp. 437-450
3. Am. J. Hum. Genet., 69(2001), pp. 1055-1061

### INDUSTRIAL APPLICABILITY

According to the present invention, there are provided a new method of detecting Paget disease of bone and a pathogenic animal with Paget disease of bone.

## Claims

1. A method for detecting Paget disease of bone by associating Paget disease of bone with the mutation of a gene coding a chondroitin synthase gene or the amount of expression of said gene.

2. The method for detecting Paget disease of bone according to claim 1, wherein the chondroitin synthase is a glycosyltransferase having activity for transferring a D-glucuronic acid residue or an N-acetyl-D-galactosamine residue to the saccharide residue at the non-reducing terminal of chondroitin.

3. The method for detecting Paget disease of bone according to claim 1 or 2, wherein the chondroitin synthase is a glycosyltransferase by means of which a xylose residue linked to an amino acid residue has D-galactose linked thereto by a β1,4-glycoside linkage.

4. The method for detecting Paget disease of bone according to claim 1, wherein the chondroitin synthase gene is a gene comprising the nucleotide sequence depicted in any one of SEQ ID NO: 1, SEQ ID NO: 3, SEQ ID NO: 5, SEQ ID NO: 65, SEQ ID NO: 67 or SEQ ID NO: 69.

5. A knockout animal wherein a glycosyltransferase gene comprising the amino acid sequence depicted in any one of SEQ ID NO: 2, SEQ ID NO: 4, SEQ ID NO: 6, SEQ ID NO: 66, SEQ ID NO: 68 or SEQ ID NO: 70 or any one of the amino acid sequences homologous thereto is partly or completely suppressed in expression.
